# EUROPEAN PATENT APPLICATION

(11) **EP 2 354 223 A1**
(43) Date of publication of application: **10.08.2011**
(21) Application number: 09814351.4
(22) Date of filing: 12.03.2009
(51) Int. Cl.: C12N 15/00

(54) **NUCLEIC ACID DRUG FOR TREATING ALLERGIC DISEASE**

(30) Priority: 16.09.2008 JP 2008237007
(71) Applicant: National University Corporation Tokyo Medical and Dental University, Bunkyo-ku Tokyo 113-8510 (JP); Haplopharma Inc., Okinawa 904-2234 (JP)
(72) Inventor: YOKOZEKI, Hiroo, Tokyo 113-8510 (JP); HOSOYA, Kazuki, Tokyo 113-8510 (JP); SATOH, Takahiro, Tokyo 113-8510 (JP); NEMOTO, Yasuhisa, Tokushima-shi Tokushima 770-0023 (JP)
(74) Representative: Keirstead, Tanis Evelyne
(86) International application number: PCT/JP2009/055383
(87) International publication number: WO 2010/032506

(57) **Abstract**

This invention provides double-stranded (ds) RNA capable of suppressing the expression of STAT6 related to allergic diseases mediated by Th2-type cytokines via RNA interference and a pharmaceutical composition for prevention or treatment of allergic diseases comprising such dsRNA. Such dsRNA is a double-stranded RNA (dsRNA) molecule targeting mRNA of the STAT6 gene composed of (a) or (b): (a) a sense strand identical to a sequence having 15 to 50 continuous nucleotides in the STAT6 gene sequence represented by SEQ ID NO: 1 or 3 and an antisense strand comprising a nucleotide sequence complementary to the nucleotide sequence of the sense strand; or (b) a sense strand comprising a sequence derived from the sequence comprising 15 to 50 continuous nucleotides of the STAT6 gene sequence represented by SEQ ID NO: 1 or 3 by deletion, substitution, or addition of 1 or several nucleotides and capable of hybridizing to the STAT6 gene and an antisense strand comprising a nucleotide sequence complementary to the nucleotide sequence of the sense strand.

SEQUENCE LISTING

<110> NATIONAL UNIVERSITY CORPORATION TOKYO MEDICAL AND DENTAL UNIVERSITY
HAPLOPHARMA INC.

<120> NUCLEIC ACID DRUG FOR TREATMENT OF ALLERGIC DISEASE

<130> TEK/FP6758734

<140> EP09814351.4
<141> 2009-03-12

<150> PCT/JP2009/055383
<151> 2009-03-12

<150> JP 2008-237007
<151> 2008-09-16

<160> 14

<170> PatentIn version 3.4

<210> 1
<211> 3993
<212> DNA
<213> Homo sapiens

<220>
<221> CDS
<222> (272)..(2815)

<400> 1
ccggaaacag cgggctgggg cagccactgc ttacactgaa gagggaggac gggagaggag 60

tgtgtgtgtg tgtgtgtgtg tgtgtgtgta tgtatgtgtg tgctttatct tatttttctt 120

tttggtggtg gtggtggaag gggggaggtg ctagcagggc cagccttgaa ctcgctggac 180

agagctacag acctatgggg cctggaagtg cccgctgaga aagggagaag acagcagagg 240

ggttgccgag gcaacctcca agtcccagat c atg tct ctg tgg ggt ctg gtc 292
Met Ser Leu Trp Gly Leu Val
1               5

tcc aag atg ccc cca gaa aaa gtg cag cgg ctc tat gtc gac ttt ccc 340
Ser Lys Met Pro Pro Glu Lys Val Gln Arg Leu Tyr Val Asp Phe Pro
        10                  15                  20

caa cac ctg cgg cat ctt ctg ggt gac tgg ctg gag agc cag ccc tgg 388
Gln His Leu Arg His Leu Leu Gly Asp Trp Leu Glu Ser Gln Pro Trp
    25                  30                  35

gag ttc ctg gtc ggc tcc gac gcc ttc tgc tgc aac ttg gct agt gcc 436
Glu Phe Leu Val Gly Ser Asp Ala Phe Cys Cys Asn Leu Ala Ser Ala
40                  45                  50                  55

cta ctt tca gac act gtc cag cac ctt cag gcc tcg gtg gga gag cag 484
Leu Leu Ser Asp Thr Val Gln His Leu Gln Ala Ser Val Gly Glu Gln
                60                  65                  70

ggg gag ggg agc acc atc ttg caa cac atc agc acc ctt gag agc ata 532
Gly Glu Gly Ser Thr Ile Leu Gln His Ile Ser Thr Leu Glu Ser Ile
            75                  80                  85

tat cag agg gac ccc ctg aag ctg gtg gcc act ttc aga caa ata ctt 580
Tyr Gln Arg Asp Pro Leu Lys Leu Val Ala Thr Phe Arg Gln Ile Leu
        90                  95                  100

caa gga gag aaa aaa gct gtt atg gaa cag ttc cgc cac ttg cca atg     628
Gln Gly Glu Lys Lys Ala Val Met Glu Gln Phe Arg His Leu Pro Met
    105                 110                 115

cct ttc cac tgg aag cag gaa gaa ctc aag ttt aag aca ggc ttg cgg     676
Pro Phe His Trp Lys Gln Glu Glu Leu Lys Phe Lys Thr Gly Leu Arg
120                 125                 130                 135

agg ctg cag cac cga gta ggg gag atc cac ctt ctc cga gaa gcc ctg     724
Arg Leu Gln His Arg Val Gly Glu Ile His Leu Leu Arg Glu Ala Leu
                140                 145                 150

cag aag ggg gct gag gct ggc caa gtg tct ctg cac agc ttg ata gaa     772
Gln Lys Gly Ala Glu Ala Gly Gln Val Ser Leu His Ser Leu Ile Glu
            155                 160                 165

act cct gct aat ggg act ggg cca agt gag gcc ctg gcc atg cta ctg     820
Thr Pro Ala Asn Gly Thr Gly Pro Ser Glu Ala Leu Ala Met Leu Leu
        170                 175                 180

cag gag acc act gga gag cta gag gca gcc aaa gcc cta gtg ctg aag     868
Gln Glu Thr Thr Gly Glu Leu Glu Ala Ala Lys Ala Leu Val Leu Lys
    185                 190                 195

agg atc cag att tgg aaa cgg cag cag cag ctg gca ggg aat ggc gca     916
Arg Ile Gln Ile Trp Lys Arg Gln Gln Gln Leu Ala Gly Asn Gly Ala
200                 205                 210                 215

ccg ttt gag gag agc ctg gcc cca ctc cag gag agg tgt gaa agc ctg     964
Pro Phe Glu Glu Ser Leu Ala Pro Leu Gln Glu Arg Cys Glu Ser Leu
                220                 225                 230

gtg gac att tat tcc cag cta cag cag gag gta ggg gcg gct ggt ggg     1012
Val Asp Ile Tyr Ser Gln Leu Gln Gln Glu Val Gly Ala Ala Gly Gly
            235                 240                 245

gag ctt gag ccc aag acc cgg gca tcg ctg act ggc cgg ctg gat gaa     1060
Glu Leu Glu Pro Lys Thr Arg Ala Ser Leu Thr Gly Arg Leu Asp Glu
        250                 255                 260

gtc ctg aga acc ctc gtc acc agt tgc ttc ctg gtg gag aag cag ccc     1108
Val Leu Arg Thr Leu Val Thr Ser Cys Phe Leu Val Glu Lys Gln Pro
    265                 270                 275

ccc cag gta ctg aag act cag acc aag ttc cag gct gga gtt cga ttc     1156
Pro Gln Val Leu Lys Thr Gln Thr Lys Phe Gln Ala Gly Val Arg Phe
280                 285                 290                 295

ctg ttg ggc ttg agg ttc ctg ggg gcc cca gcc aag cct ccg ctg gtc     1204
Leu Leu Gly Leu Arg Phe Leu Gly Ala Pro Ala Lys Pro Pro Leu Val
                300                 305                 310

agg gcc gac atg gtg aca gag aag cag gcg cgg gag ctg agt gtg cct     1252
Arg Ala Asp Met Val Thr Glu Lys Gln Ala Arg Glu Leu Ser Val Pro
            315                 320                 325

cag ggt cct ggg gct gga gca gaa agc act gga gaa atc atc aac aac     1300
Gln Gly Pro Gly Ala Gly Ala Glu Ser Thr Gly Glu Ile Ile Asn Asn
        330                 335                 340

act gtg ccc ttg gag aac agc att cct ggg aac tgc tgc tct gcc ctg     1348
Thr Val Pro Leu Glu Asn Ser Ile Pro Gly Asn Cys Cys Ser Ala Leu
    345                 350                 355

ttc aag aac ctg ctt ctc aag aag atc aag cgg tgt gag cgg aag ggc     1396
Phe Lys Asn Leu Leu Leu Lys Lys Ile Lys Arg Cys Glu Arg Lys Gly
360                 365                 370                 375

act gag tct gtc aca gag gag aag tgc gct gtg ctc ttc tct gcc agc 1444
Thr Glu Ser Val Thr Glu Glu Lys Cys Ala Val Leu Phe Ser Ala Ser
                380                 385                 390

ttc aca ctt ggc ccc ggc aaa ctc ccc atc cag ctc cag gcc ctg tct 1492
Phe Thr Leu Gly Pro Gly Lys Leu Pro Ile Gln Leu Gln Ala Leu Ser
            395                 400                 405

ctg ccc ctg gtg gtc atc gtc cat ggc aac caa gac aac aat gcc aaa 1540
Leu Pro Leu Val Val Ile Val His Gly Asn Gln Asp Asn Asn Ala Lys
        410                 415                 420

gcc act atc ctg tgg gac aat gcc ttc tct gag atg gac cgc gtg ccc 1588
Ala Thr Ile Leu Trp Asp Asn Ala Phe Ser Glu Met Asp Arg Val Pro
    425                 430                 435

ttt gtg gtg gct gag cgg gtg ccc tgg gag aag atg tgt gaa act ctg 1636
Phe Val Val Ala Glu Arg Val Pro Trp Glu Lys Met Cys Glu Thr Leu
440                 445                 450                 455

aac ctg aag ttc atg gct gag gtg ggg acc aac cgg ggg ctg ctc cca 1684
Asn Leu Lys Phe Met Ala Glu Val Gly Thr Asn Arg Gly Leu Leu Pro
                460                 465                 470

gag cac ttc ctc ttc ctg gcc cag aag atc ttc aat gac aac agc ctc 1732
Glu His Phe Leu Phe Leu Ala Gln Lys Ile Phe Asn Asp Asn Ser Leu
            475                 480                 485

agt atg gag gcc ttc cag cac cgt tct gtg tcc tgg tcg cag ttc aac 1780
Ser Met Glu Ala Phe Gln His Arg Ser Val Ser Trp Ser Gln Phe Asn
        490                 495                 500

aag gag atc ctg ctg ggc cgt ggc ttc acc ttt tgg cag tgg ttt gat 1828
Lys Glu Ile Leu Leu Gly Arg Gly Phe Thr Phe Trp Gln Trp Phe Asp
    505                 510                 515

ggt gtc ctg gac ctc acc aaa cgc tgt ctc cgg agc tac tgg tct gac 1876
Gly Val Leu Asp Leu Thr Lys Arg Cys Leu Arg Ser Tyr Trp Ser Asp
520                 525                 530                 535

cgg ctg atc att ggc ttc atc agc aaa cag tac gtt act agc ctt ctt 1924
Arg Leu Ile Ile Gly Phe Ile Ser Lys Gln Tyr Val Thr Ser Leu Leu
                540                 545                 550

ctc aat gag ccc gac gga acc ttt ctc ctc cgc ttc agc gac tca gag 1972
Leu Asn Glu Pro Asp Gly Thr Phe Leu Leu Arg Phe Ser Asp Ser Glu
            555                 560                 565

att ggg ggc atc acc att gcc cat gtc atc cgg ggc cag gat ggc tct 2020
Ile Gly Gly Ile Thr Ile Ala His Val Ile Arg Gly Gln Asp Gly Ser
        570                 575                 580

cca cag ata gag aac atc cag cca ttc tct gcc aaa gac ctg tcc att 2068
Pro Gln Ile Glu Asn Ile Gln Pro Phe Ser Ala Lys Asp Leu Ser Ile
    585                 590                 595

cgc tca ctg ggg gac cga atc cgg gat ctt gct cag ctc aaa aat ctc 2116
Arg Ser Leu Gly Asp Arg Ile Arg Asp Leu Ala Gln Leu Lys Asn Leu
600                 605                 610                 615

tat ccc aag aag ccc aag gat gag gct ttc cgg agc cac tac aag cct 2164
Tyr Pro Lys Lys Pro Lys Asp Glu Ala Phe Arg Ser His Tyr Lys Pro
                620                 625                 630

gaa cag atg ggt aag gat ggc agg ggt tat gtc cca gct acc atc aag 2212
Glu Gln Met Gly Lys Asp Gly Arg Gly Tyr Val Pro Ala Thr Ile Lys
            635                 640                 645

atg acc gtg gaa agg gac caa cca ctt cct acc cca gag ctc cag atg     2260
Met Thr Val Glu Arg Asp Gln Pro Leu Pro Thr Pro Glu Leu Gln Met
        650                 655                 660

cct acc atg gtg cct tct tat gac ctt gga atg gcc cct gat tcc tcc     2308
Pro Thr Met Val Pro Ser Tyr Asp Leu Gly Met Ala Pro Asp Ser Ser
    665                 670                 675

atg agc atg cag ctt ggc cca gat atg gtg ccc cag gtg tac cca cca     2356
Met Ser Met Gln Leu Gly Pro Asp Met Val Pro Gln Val Tyr Pro Pro
680                 685                 690                 695

cac tct cac tcc atc ccc ccg tat caa ggc ctc tcc cca gaa gaa tca     2404
His Ser His Ser Ile Pro Pro Tyr Gln Gly Leu Ser Pro Glu Glu Ser
                700                 705                 710

gtc aac gtg ttg tca gcc ttc cag gag cct cac ctg cag atg ccc ccc     2452
Val Asn Val Leu Ser Ala Phe Gln Glu Pro His Leu Gln Met Pro Pro
            715                 720                 725

agc ctg ggc cag atg agc ctg ccc ttt gac cag cct cac ccc cag ggc     2500
Ser Leu Gly Gln Met Ser Leu Pro Phe Asp Gln Pro His Pro Gln Gly
        730                 735                 740

ctg ctg ccg tgc cag cct cag gag cat gct gtg tcc agc cct gac ccc     2548
Leu Leu Pro Cys Gln Pro Gln Glu His Ala Val Ser Ser Pro Asp Pro
    745                 750                 755

ctg ctc tgc tca gat gtg acc atg gtg gaa gac agc tgc ctg agc cag     2596
Leu Leu Cys Ser Asp Val Thr Met Val Glu Asp Ser Cys Leu Ser Gln
760                 765                 770                 775

cca gtg aca gcg ttt cct cag ggc act tgg att ggt gaa gac ata ttc     2644
Pro Val Thr Ala Phe Pro Gln Gly Thr Trp Ile Gly Glu Asp Ile Phe
                780                 785                 790

cct cct ctg ctg cct ccc act gaa cag gac ctc act aag ctt ctc ctg     2692
Pro Pro Leu Leu Pro Pro Thr Glu Gln Asp Leu Thr Lys Leu Leu Leu
            795                 800                 805

gag ggg caa ggg gag tcg ggg gga ggg tcc ttg ggg gca cag ccc ctc     2740
Glu Gly Gln Gly Glu Ser Gly Gly Gly Ser Leu Gly Ala Gln Pro Leu
        810                 815                 820

ctg cag ccc tcc cac tat ggg caa tct ggg atc tca atg tcc cac atg     2788
Leu Gln Pro Ser His Tyr Gly Gln Ser Gly Ile Ser Met Ser His Met
    825                 830                 835

gac cta agg gcc aac ccc agt tgg tga tcccagctgg agggagaacc           2835
Asp Leu Arg Ala Asn Pro Ser Trp
840                 845

caaagagaca gctcttctac tacccccaca gacctgctct ggacacttgc tcatgccctg   2895

ccaagcagca gatggggagg gtgccctcct atccccacct actcctgggt caggaggaaa   2955

agactaacag gagaatgcac agtgggtgga gccaatccac tccttccttt ctatcattcc   3015

cctgcccacc tccttccagc actgactgga agggaagttc aggctctgag acacgcccca   3075

acatgcctgc acctgcagcg cgcacacgca cgcacacaca catacagagc tctctgaggg   3135

tgatggggct gagcaggagg ggggctgggt aagagcacag gttagggcat ggaaggcttc   3195

tccgcccatt ctgacccagg gcctaggacg gataggcagg aacatacaga cacatttaca   3255

ctagaggcca gggatagagg atattgggtc tcagccctag gggaatggga agcagctcaa   3315

gggaccctgg gtgggagcat aggaggggtc tggacatgtg gttactagta caggttttgc 3375

cctgattaaa aaatctccca aagccccaaa ttcctgttag ccaggtggag gcttctgata 3435

cgtgtatgag actatgcaaa agtacaaggg ctgagattct tcgtgtatag ctgtgtgaac 3495

gtgtatgtac ctaggatatg ttaaatgtat agctggcacc ttagttgcat gaccacatag 3555

aacatgtgtc tatctgcttt tgcctacgtg acaacacaaa tttgggaggg tgagacactg 3615

cacagaagac agcagcaagt gtgctggcct ctctgacata tgctaacccc caaatactct 3675

gaatttggag tctgactgtg cccaagtggg tccaagtggc tgtgacatct acgtatggct 3735

ccacacctcc aatgctgcct gggagccagg gtgagagtct gggtccaggc ctggccatgt 3795

ggccctccag tgtatgagag ggccctgcct gctgcatctt ttctgttgcc ccatccaccg 3855

ccagcttccc ttcactcccc tatcccattc tccctctcaa ggcaggggtc atagatccta 3915

agccataaaa taaattttat tccaaaataa caaaataaat aatctactgt acacaatctg 3975

aaaaaaaaaa aaaaaaaa 3993

<210> 2
<211> 847
<212> PRT
<213> Homo sapiens

<400> 2

Met Ser Leu Trp Gly Leu Val Ser Lys Met Pro Pro Glu Lys Val Gln
1               5                   10                  15

Arg Leu Tyr Val Asp Phe Pro Gln His Leu Arg His Leu Leu Gly Asp
            20                  25                  30

Trp Leu Glu Ser Gln Pro Trp Glu Phe Leu Val Gly Ser Asp Ala Phe
        35                  40                  45

Cys Cys Asn Leu Ala Ser Ala Leu Leu Ser Asp Thr Val Gln His Leu
    50                  55                  60

Gln Ala Ser Val Gly Glu Gln Gly Glu Gly Ser Thr Ile Leu Gln His
65                  70                  75                  80

Ile Ser Thr Leu Glu Ser Ile Tyr Gln Arg Asp Pro Leu Lys Leu Val
                85                  90                  95

Ala Thr Phe Arg Gln Ile Leu Gln Gly Glu Lys Lys Ala Val Met Glu
            100                 105                 110

Gln Phe Arg His Leu Pro Met Pro Phe His Trp Lys Gln Glu Glu Leu
        115                 120                 125

Lys Phe Lys Thr Gly Leu Arg Arg Leu Gln His Arg Val Gly Glu Ile
    130                 135                 140

His Leu Leu Arg Glu Ala Leu Gln Lys Gly Ala Glu Ala Gly Gln Val
145                 150                 155                 160

Ser Leu His Ser Leu Ile Glu Thr Pro Ala Asn Gly Thr Gly Pro Ser
                165                 170                 175

Glu Ala Leu Ala Met Leu Leu Gln Glu Thr Thr Gly Glu Leu Glu Ala
            180                 185                 190

Ala Lys Ala Leu Val Leu Lys Arg Ile Gln Ile Trp Lys Arg Gln Gln
        195                 200                 205

Gln Leu Ala Gly Asn Gly Ala Pro Phe Glu Glu Ser Leu Ala Pro Leu
    210                 215                 220

Gln Glu Arg Cys Glu Ser Leu Val Asp Ile Tyr Ser Gln Leu Gln Gln
225                 230                 235                 240

Glu Val Gly Ala Ala Gly Gly Glu Leu Glu Pro Lys Thr Arg Ala Ser
                245                 250                 255

Leu Thr Gly Arg Leu Asp Glu Val Leu Arg Thr Leu Val Thr Ser Cys
            260                 265                 270

Phe Leu Val Glu Lys Gln Pro Pro Gln Val Leu Lys Thr Gln Thr Lys
        275                 280                 285

Phe Gln Ala Gly Val Arg Phe Leu Leu Gly Leu Arg Phe Leu Gly Ala
    290                 295                 300

Pro Ala Lys Pro Pro Leu Val Arg Ala Asp Met Val Thr Glu Lys Gln
305                 310                 315                 320

Ala Arg Glu Leu Ser Val Pro Gln Gly Pro Gly Ala Gly Ala Glu Ser
                325                 330                 335

Thr Gly Glu Ile Ile Asn Asn Thr Val Pro Leu Glu Asn Ser Ile Pro
            340                 345                 350

Gly Asn Cys Cys Ser Ala Leu Phe Lys Asn Leu Leu Leu Lys Lys Ile
        355                 360                 365

Lys Arg Cys Glu Arg Lys Gly Thr Glu Ser Val Thr Glu Glu Lys Cys
    370                 375                 380

Ala Val Leu Phe Ser Ala Ser Phe Thr Leu Gly Pro Gly Lys Leu Pro
385                 390                 395                 400

Ile Gln Leu Gln Ala Leu Ser Leu Pro Leu Val Val Ile Val His Gly
                405                 410                 415

Asn Gln Asp Asn Asn Ala Lys Ala Thr Ile Leu Trp Asp Asn Ala Phe
            420                 425                 430

Ser Glu Met Asp Arg Val Pro Phe Val Val Ala Glu Arg Val Pro Trp
        435                 440                 445

Glu Lys Met Cys Glu Thr Leu Asn Leu Lys Phe Met Ala Glu Val Gly
    450                 455                 460

Thr Asn Arg Gly Leu Leu Pro Glu His Phe Leu Phe Leu Ala Gln Lys
465                 470                 475                 480

Ile Phe Asn Asp Asn Ser Leu Ser Met Glu Ala Phe Gln His Arg Ser
                485                 490                 495

Val Ser Trp Ser Gln Phe Asn Lys Glu Ile Leu Leu Gly Arg Gly Phe
            500                 505                 510

Thr Phe Trp Gln Trp Phe Asp Gly Val Leu Asp Leu Thr Lys Arg Cys
        515                 520                 525

Leu Arg Ser Tyr Trp Ser Asp Arg Leu Ile Ile Gly Phe Ile Ser Lys
    530                 535                 540

Gln Tyr Val Thr Ser Leu Leu Leu Asn Glu Pro Asp Gly Thr Phe Leu
545                 550                 555                 560

Leu Arg Phe Ser Asp Ser Glu Ile Gly Gly Ile Thr Ile Ala His Val
                565                 570                 575

Ile Arg Gly Gln Asp Gly Ser Pro Gln Ile Glu Asn Ile Gln Pro Phe
            580                 585                 590

Ser Ala Lys Asp Leu Ser Ile Arg Ser Leu Gly Asp Arg Ile Arg Asp
        595                 600                 605

Leu Ala Gln Leu Lys Asn Leu Tyr Pro Lys Lys Pro Lys Asp Glu Ala
    610                 615                 620

Phe Arg Ser His Tyr Lys Pro Glu Gln Met Gly Lys Asp Gly Arg Gly
625                 630                 635                 640

Tyr Val Pro Ala Thr Ile Lys Met Thr Val Glu Arg Asp Gln Pro Leu
                645                 650                 655

Pro Thr Pro Glu Leu Gln Met Pro Thr Met Val Pro Ser Tyr Asp Leu
            660                 665                 670

Gly Met Ala Pro Asp Ser Ser Met Ser Met Gln Leu Gly Pro Asp Met
        675                 680                 685

Val Pro Gln Val Tyr Pro Pro His Ser His Ser Ile Pro Pro Tyr Gln
    690                 695                 700

Gly Leu Ser Pro Glu Glu Ser Val Asn Val Leu Ser Ala Phe Gln Glu
705                 710                 715                 720

Pro His Leu Gln Met Pro Pro Ser Leu Gly Gln Met Ser Leu Pro Phe
                725                 730                 735

Asp Gln Pro His Pro Gln Gly Leu Leu Pro Cys Gln Pro Gln Glu His
            740                 745                 750

Ala Val Ser Ser Pro Asp Pro Leu Leu Cys Ser Asp Val Thr Met Val
        755                 760                 765

Glu Asp Ser Cys Leu Ser Gln Pro Val Thr Ala Phe Pro Gln Gly Thr
    770                 775                 780

Trp Ile Gly Glu Asp Ile Phe Pro Pro Leu Leu Pro Pro Thr Glu Gln
785                 790                 795                 800

Asp Leu Thr Lys Leu Leu Leu Glu Gly Gln Gly Glu Ser Gly Gly Gly
                805                 810                 815

Ser Leu Gly Ala Gln Pro Leu Leu Gln Pro Ser His Tyr Gly Gln Ser
            820                 825                 830

Gly Ile Ser Met Ser His Met Asp Leu Arg Ala Asn Pro Ser Trp
        835                 840                 845

<210>  3
<211>  3805
<212>  DNA
<213>  Mus musculus

<220>
<221>  CDS
<222>  (286)..(2799)

<400>  3
gagaagccgg aaacagcagg ccggggcagc cagggtttac agtgaagaag gcccggagac      60

gagtgcgtgc gtgcctgtgt gtgtgtttgt gtgtgtgtgc gcgcgctcga gcgtgtgcgc     120

gcgtgcctgt gtgcatatgt gtgtgtgtgt ctgtacacat tgagttttta gggccagccc     180

aggacccgct ggacagacct acagacccat ggggcttggt agtgccctct gagagaggga     240

gaagatagca gcggggctgc tgaggcaccc tgtatatccc agatc atg tct ctg tgg     297
                                                  Met Ser Leu Trp
                                                  1

ggc cta att tcc aag atg tcc cca gaa aaa ctg caa cgg ctc tat gtt       345
Gly Leu Ile Ser Lys Met Ser Pro Glu Lys Leu Gln Arg Leu Tyr Val
5                   10                  15                  20

gac ttt cca caa cgc cta cgg cat ctc ctg gct gac tgg ctg gag agc 393
Asp Phe Pro Gln Arg Leu Arg His Leu Leu Ala Asp Trp Leu Glu Ser
                25                  30                  35

cag ccc tgg gag ttc ctg gtc ggt tca gat gct ttc tgt tac aac atg 441
Gln Pro Trp Glu Phe Leu Val Gly Ser Asp Ala Phe Cys Tyr Asn Met
            40                  45                  50

gcc agt gcc cta ctt tct gcc aca gtc cag cgt ctt cag gcc act gct 489
Ala Ser Ala Leu Leu Ser Ala Thr Val Gln Arg Leu Gln Ala Thr Ala
        55                  60                  65

gga gag cag ggg aag gga aac agc atc ttg ccg cac atc agc acc ttg 537
Gly Glu Gln Gly Lys Gly Asn Ser Ile Leu Pro His Ile Ser Thr Leu
    70                  75                  80

gag agc atc tat cag agg gac ccc ctg aag ctg gtg gcc acc atc aga 585
Glu Ser Ile Tyr Gln Arg Asp Pro Leu Lys Leu Val Ala Thr Ile Arg
85                  90                  95                  100

caa ata ctt caa ggg gag aaa aaa gct gtt ata gaa gag ttc cgc cac 633
Gln Ile Leu Gln Gly Glu Lys Lys Ala Val Ile Glu Glu Phe Arg His
                105                 110                 115

ctg cca ggg ccc ttc cat cgg aag cag gaa gaa ctc aag ttt act aca 681
Leu Pro Gly Pro Phe His Arg Lys Gln Glu Glu Leu Lys Phe Thr Thr
            120                 125                 130

gcc ctc gga agg ctt cag cat cga gta agg gag acc cgg ctt ctc cga 729
Ala Leu Gly Arg Leu Gln His Arg Val Arg Glu Thr Arg Leu Leu Arg
        135                 140                 145

gaa tct ctg cag caa ggg gct aag act gga caa gtg tct ctg cag aat 777
Glu Ser Leu Gln Gln Gly Ala Lys Thr Gly Gln Val Ser Leu Gln Asn
    150                 155                 160

ttg ata gac cct cct gtc aat ggt cct ggt cca agt gag gac ctg gcc 825
Leu Ile Asp Pro Pro Val Asn Gly Pro Gly Pro Ser Glu Asp Leu Ala
165                 170                 175                 180

acc atg ctg cag ggg act gtg ggg gac ctg gag gcc acc cag gcc ctg 873
Thr Met Leu Gln Gly Thr Val Gly Asp Leu Glu Ala Thr Gln Ala Leu
                185                 190                 195

gtg ctg aaa agg att cag att tgg aag cgg cag caa cag ctg gca ggg 921
Val Leu Lys Arg Ile Gln Ile Trp Lys Arg Gln Gln Gln Leu Ala Gly
            200                 205                 210

aat ggc aca ccc ttt gag gag agc cta gca ggg ctg cag gag agg tgt 969
Asn Gly Thr Pro Phe Glu Glu Ser Leu Ala Gly Leu Gln Glu Arg Cys
        215                 220                 225

gaa agc ctg gtg gaa att tat tcc cag ctg cag cag gag att ggg gca 1017
Glu Ser Leu Val Glu Ile Tyr Ser Gln Leu Gln Gln Glu Ile Gly Ala
    230                 235                 240

gcc agt gga gaa ctg gaa ccc aag acc cgg gca tcg ctg ata agc cgt 1065
Ala Ser Gly Glu Leu Glu Pro Lys Thr Arg Ala Ser Leu Ile Ser Arg
245                 250                 255                 260

ctg gat gaa gtc ctg cga acc ctt gtg acc agc tct ttc ctg gtg gag 1113
Leu Asp Glu Val Leu Arg Thr Leu Val Thr Ser Ser Phe Leu Val Glu
                265                 270                 275

aag cag ccc ccg cag gtt ctg aag aca cag act aag ttc cag gct ggg 1161
Lys Gln Pro Pro Gln Val Leu Lys Thr Gln Thr Lys Phe Gln Ala Gly
            280                 285                 290

gtt cga ttc ctg ctg ggt ctg cag ttt cta ggg acc tca gcc aag cct 1209
Val Arg Phe Leu Leu Gly Leu Gln Phe Leu Gly Thr Ser Ala Lys Pro
        295                 300                 305

cca atg gtc aga gct gac atg gtg aca gag aaa cag gcc aga gaa cta 1257
Pro Met Val Arg Ala Asp Met Val Thr Glu Lys Gln Ala Arg Glu Leu
    310                 315                 320

agc ctg gcc cag ggg ccc ggg act gga gtg gag agc aca gga gag atc 1305
Ser Leu Ala Gln Gly Pro Gly Thr Gly Val Glu Ser Thr Gly Glu Ile
325                 330                 335                 340

atg aac aac acg gtg ccc ctg gag aac agc att ccc agc aac tgc tgc 1353
Met Asn Asn Thr Val Pro Leu Glu Asn Ser Ile Pro Ser Asn Cys Cys
                345                 350                 355

tcc gcc ctg ttc aag aac ctg ctc ctg aag aaa ata aag cgc tgt gag 1401
Ser Ala Leu Phe Lys Asn Leu Leu Leu Lys Lys Ile Lys Arg Cys Glu
            360                 365                 370

cgg aag ggc aca gag tct gtc acc gag gag aag tgt gct gtg ctc ttc 1449
Arg Lys Gly Thr Glu Ser Val Thr Glu Glu Lys Cys Ala Val Leu Phe
        375                 380                 385

tcc acg agc ttc aca ttg ggc ccc aac aaa ctt ctc atc cag ctt cag 1497
Ser Thr Ser Phe Thr Leu Gly Pro Asn Lys Leu Leu Ile Gln Leu Gln
    390                 395                 400

gcc ctg tct ctg ccc ttg gtg gtc atc gtg cat ggt aac caa gac aac 1545
Ala Leu Ser Leu Pro Leu Val Val Ile Val His Gly Asn Gln Asp Asn
405                 410                 415                 420

aac gcc aaa gct acc atc cta tgg gac aat gcc ttc tct gag atg gac 1593
Asn Ala Lys Ala Thr Ile Leu Trp Asp Asn Ala Phe Ser Glu Met Asp
                425                 430                 435

cga gtg ccc ttt gtg gtg gct gag cga gtg ccc tgg gag aag atg tgt 1641
Arg Val Pro Phe Val Val Ala Glu Arg Val Pro Trp Glu Lys Met Cys
            440                 445                 450

gaa acc cta aac ctc aag ttt atg gct gag gtg ggg acc agc cgg gga 1689
Glu Thr Leu Asn Leu Lys Phe Met Ala Glu Val Gly Thr Ser Arg Gly
        455                 460                 465

ctg ctt cca gag cac ttc ctg ttc ctc gcc cag aag atc ttc aac gac 1737
Leu Leu Pro Glu His Phe Leu Phe Leu Ala Gln Lys Ile Phe Asn Asp
    470                 475                 480

aac agc ctc agt gtg gag gcc ttt cag cac cgc tgt gtg tcc tgg tca 1785
Asn Ser Leu Ser Val Glu Ala Phe Gln His Arg Cys Val Ser Trp Ser
485                 490                 495                 500

cag ttc aat aag gag atc ctg ctg ggc cga ggc ttc aca ttt tgg cag 1833
Gln Phe Asn Lys Glu Ile Leu Leu Gly Arg Gly Phe Thr Phe Trp Gln
                505                 510                 515

tgg ttt gat ggt gtc ctg gac ctc acc aaa cgc tgt ctc cgg agc tac 1881
Trp Phe Asp Gly Val Leu Asp Leu Thr Lys Arg Cys Leu Arg Ser Tyr
            520                 525                 530

tgg tca gat cgg ctg atc att ggc ttt att agt aag caa tat gtc act 1929
Trp Ser Asp Arg Leu Ile Ile Gly Phe Ile Ser Lys Gln Tyr Val Thr
        535                 540                 545

agc ctt ctc ctc aat gag cca gat ggg acc ttc ctc ctc cgc ttt agc 1977
Ser Leu Leu Leu Asn Glu Pro Asp Gly Thr Phe Leu Leu Arg Phe Ser
    550                 555                 560

gac tct gag atc ggg ggc atc acc att gca cac gtc atc cgg ggt cag 2025
Asp Ser Glu Ile Gly Gly Ile Thr Ile Ala His Val Ile Arg Gly Gln
565                 570                 575                 580

gat ggc tcc tca cag ata gag aac atc cag cca ttt tct gcc aaa gac 2073
Asp Gly Ser Ser Gln Ile Glu Asn Ile Gln Pro Phe Ser Ala Lys Asp
                585                 590                 595

ctg tcc att cgc tca ctg ggg gac cgg atc cgg gat ctt gct cag tta 2121
Leu Ser Ile Arg Ser Leu Gly Asp Arg Ile Arg Asp Leu Ala Gln Leu
            600                 605                 610

aaa aac ctc tac ccc aag aaa ccc aaa gat gag gct ttc cgg agt cac 2169
Lys Asn Leu Tyr Pro Lys Lys Pro Lys Asp Glu Ala Phe Arg Ser His
        615                 620                 625

tat aag ccc gaa cag atg ggg aag gac ggg agg ggt tat gtc tct act 2217
Tyr Lys Pro Glu Gln Met Gly Lys Asp Gly Arg Gly Tyr Val Ser Thr
    630                 635                 640

act atc aag atg act gtg gaa agg gac cag ccc ctt cct act cca gag 2265
Thr Ile Lys Met Thr Val Glu Arg Asp Gln Pro Leu Pro Thr Pro Glu
645                 650                 655                 660

ccc cag atg cct gcc atg gtg cca cct tat gat ctt gga atg gcc cct 2313
Pro Gln Met Pro Ala Met Val Pro Pro Tyr Asp Leu Gly Met Ala Pro
                665                 670                 675

gat gct tcc atg caa ctc agc tca gat atg ggg tat cct cca cag tcc 2361
Asp Ala Ser Met Gln Leu Ser Ser Asp Met Gly Tyr Pro Pro Gln Ser
            680                 685                 690

atc cac tca ttt cag agc cta gaa gag tcc atg agt gta ctg cca tct 2409
Ile His Ser Phe Gln Ser Leu Glu Glu Ser Met Ser Val Leu Pro Ser
        695                 700                 705

ttt cag gag cct cac ctg caa atg ccc ccc aac atg agc cag ata acc 2457
Phe Gln Glu Pro His Leu Gln Met Pro Pro Asn Met Ser Gln Ile Thr
    710                 715                 720

atg ccc ttt gac cag cct cac ccc cag ggt ctg ctg cag tgc cag tcc 2505
Met Pro Phe Asp Gln Pro His Pro Gln Gly Leu Leu Gln Cys Gln Ser
725                 730                 735                 740

cag gaa cat gct gtg tcc agc cct gaa ccc atg ctt tgc tca gat gtg 2553
Gln Glu His Ala Val Ser Ser Pro Glu Pro Met Leu Cys Ser Asp Val
                745                 750                 755

act atg gta gag gac agc tgc cta act cag cct gtg gga ggt ttc ccc 2601
Thr Met Val Glu Asp Ser Cys Leu Thr Gln Pro Val Gly Gly Phe Pro
            760                 765                 770

caa ggc acc tgg gtc agt gaa gac atg tac cct ccc ctg atg cct ccc 2649
Gln Gly Thr Trp Val Ser Glu Asp Met Tyr Pro Pro Leu Met Pro Pro
        775                 780                 785

act gaa cag gac ctc acc aag ctt ctc ctg gag aac caa ggg gag gca 2697
Thr Glu Gln Asp Leu Thr Lys Leu Leu Leu Glu Asn Gln Gly Glu Ala
    790                 795                 800

gga ggg tcc tta gga agc cag ccc ctc ctg caa cca tct cct tat ggg 2745
Gly Gly Ser Leu Gly Ser Gln Pro Leu Leu Gln Pro Ser Pro Tyr Gly
805                 810                 815                 820

caa tca ggg atc tca ctg tcc cac ctg gac cta agg acc aac ccc agc 2793
Gln Ser Gly Ile Ser Leu Ser His Leu Asp Leu Arg Thr Asn Pro Ser
                825                 830                 835

tgg tga tcccagctgg agaagcccag aaacaaagcc tcttctgtct ctatggacca     2849
Trp

gctctggaca cctgctcatg caggtgcctt ccgtctcaac tgttccttgg tcaagagaaa     2909

agaactggct gggagaccat gtggtgtatg gaactgctgt gctctgtcct tcctgccata     2969

tcagggcccc ccttttccag cactgggtgc aaagggatga gtggggtgtt aatgctcgaa     3029

tgtgatacaa ctgtatcaca acacacacgc acacacacat acacacacac cagaactgtg     3089

ttgagccagg gcctgggact caacatacag aaacatagag acattgtgcc aaagacagag     3149

ggcatatagg cctagggcat tgaagctggg ctcaggtgac tctgggaggg agaaaaagga     3209

aaaagtgggt atacagtcac tggtgtgagt tctacccaga ttttaaaaaa caagactcca     3269

aagctccaaa ttcttgcaaa aaaagatgcc tagtgacatt tgagactgca ttctaagagc     3329

taagcttgtg tatagctgta ccaatgttta cccaagacat gttaacctat agaagtcaca     3389

catcactgta tgaccgcaca gaacatgtat cttctgcttt tgccagtgtg accttaacat     3449

atctgaaagg ctgagacatt gtataagaca gcaacccagt atcatttggg gagtaactat     3509

gtggctgtga catgcataaa gctctagcct gggtaacttg atgcttccag tgtttcctag     3569

agcctgggat ataagtaggg atgcagacct ctctgtgtaa actccctggg cgttgaggcc     3629

ctcaactgct gtctcttgta cttttctgtc cactgccagc tctgcccccc tcccccggct     3689

cttacccagt cctttttccc tcactggagg ggaagggggc catggatcct aagccataaa     3749

ataaatttta ttccaaaata acaaaataaa taatctactg tacacaatct gaaaag         3805

<210>  4
<211>  837
<212>  PRT
<213>  Mus musculus

<400>  4

Met Ser Leu Trp Gly Leu Ile Ser Lys Met Ser Pro Glu Lys Leu Gln
1               5                   10                  15

Arg Leu Tyr Val Asp Phe Pro Gln Arg Leu Arg His Leu Leu Ala Asp
            20                  25                  30

Trp Leu Glu Ser Gln Pro Trp Glu Phe Leu Val Gly Ser Asp Ala Phe
        35                  40                  45

Cys Tyr Asn Met Ala Ser Ala Leu Leu Ser Ala Thr Val Gln Arg Leu
    50                  55                  60

Gln Ala Thr Ala Gly Glu Gln Gly Lys Gly Asn Ser Ile Leu Pro His
65                  70                  75                  80

Ile Ser Thr Leu Glu Ser Ile Tyr Gln Arg Asp Pro Leu Lys Leu Val
                85                  90                  95

Ala Thr Ile Arg Gln Ile Leu Gln Gly Glu Lys Lys Ala Val Ile Glu
            100                 105                 110

Glu Phe Arg His Leu Pro Gly Pro Phe His Arg Lys Gln Glu Glu Leu
        115                 120                 125

Lys Phe Thr Thr Ala Leu Gly Arg Leu Gln His Arg Val Arg Glu Thr
    130                 135                 140

Arg Leu Leu Arg Glu Ser Leu Gln Gln Gly Ala Lys Thr Gly Gln Val
145                 150                 155                 160

Ser Leu Gln Asn Leu Ile Asp Pro Pro Val Asn Gly Pro Gly Pro Ser
                165                 170                 175

Glu Asp Leu Ala Thr Met Leu Gln Gly Thr Val Gly Asp Leu Glu Ala
            180                 185                 190

Thr Gln Ala Leu Val Leu Lys Arg Ile Gln Ile Trp Lys Arg Gln Gln
        195                 200                 205

Gln Leu Ala Gly Asn Gly Thr Pro Phe Glu Glu Ser Leu Ala Gly Leu
    210                 215                 220

Gln Glu Arg Cys Glu Ser Leu Val Glu Ile Tyr Ser Gln Leu Gln Gln
225                 230                 235                 240

Glu Ile Gly Ala Ala Ser Gly Glu Leu Glu Pro Lys Thr Arg Ala Ser
                245                 250                 255

Leu Ile Ser Arg Leu Asp Glu Val Leu Arg Thr Leu Val Thr Ser Ser
            260                 265                 270

Phe Leu Val Glu Lys Gln Pro Pro Gln Val Leu Lys Thr Gln Thr Lys
        275                 280                 285

Phe Gln Ala Gly Val Arg Phe Leu Leu Gly Leu Gln Phe Leu Gly Thr
    290                 295                 300

Ser Ala Lys Pro Pro Met Val Arg Ala Asp Met Val Thr Glu Lys Gln
305                 310                 315                 320

Ala Arg Glu Leu Ser Leu Ala Gln Gly Pro Gly Thr Gly Val Glu Ser
                325                 330                 335

Thr Gly Glu Ile Met Asn Asn Thr Val Pro Leu Glu Asn Ser Ile Pro
            340                 345                 350

Ser Asn Cys Cys Ser Ala Leu Phe Lys Asn Leu Leu Leu Lys Lys Ile
        355                 360                 365

Lys Arg Cys Glu Arg Lys Gly Thr Glu Ser Val Thr Glu Glu Lys Cys
    370                 375                 380

Ala Val Leu Phe Ser Thr Ser Phe Thr Leu Gly Pro Asn Lys Leu Leu
385                 390                 395                 400

Ile Gln Leu Gln Ala Leu Ser Leu Pro Leu Val Val Ile Val His Gly
                405                 410                 415

Asn Gln Asp Asn Asn Ala Lys Ala Thr Ile Leu Trp Asp Asn Ala Phe
            420                 425                 430

Ser Glu Met Asp Arg Val Pro Phe Val Val Ala Glu Arg Val Pro Trp
        435                 440                 445

Glu Lys Met Cys Glu Thr Leu Asn Leu Lys Phe Met Ala Glu Val Gly
    450                 455                 460

Thr Ser Arg Gly Leu Leu Pro Glu His Phe Leu Phe Leu Ala Gln Lys
465                 470                 475                 480

Ile Phe Asn Asp Asn Ser Leu Ser Val Glu Ala Phe Gln His Arg Cys
                485                 490                 495

Val Ser Trp Ser Gln Phe Asn Lys Glu Ile Leu Leu Gly Arg Gly Phe
            500                 505                 510

Thr Phe Trp Gln Trp Phe Asp Gly Val Leu Asp Leu Thr Lys Arg Cys
        515                 520                 525

Leu Arg Ser Tyr Trp Ser Asp Arg Leu Ile Ile Gly Phe Ile Ser Lys
    530                 535                 540

Gln Tyr Val Thr Ser Leu Leu Leu Asn Glu Pro Asp Gly Thr Phe Leu
545                 550                 555                 560

Leu Arg Phe Ser Asp Ser Glu Ile Gly Gly Ile Thr Ile Ala His Val
                565                 570                 575

Ile Arg Gly Gln Asp Gly Ser Ser Gln Ile Glu Asn Ile Gln Pro Phe
            580                 585                 590

Ser Ala Lys Asp Leu Ser Ile Arg Ser Leu Gly Asp Arg Ile Arg Asp
        595                 600                 605

Leu Ala Gln Leu Lys Asn Leu Tyr Pro Lys Lys Pro Lys Asp Glu Ala
    610                 615                 620

Phe Arg Ser His Tyr Lys Pro Glu Gln Met Gly Lys Asp Gly Arg Gly
625                 630                 635                 640

Tyr Val Ser Thr Thr Ile Lys Met Thr Val Glu Arg Asp Gln Pro Leu
                645                 650                 655

Pro Thr Pro Glu Pro Gln Met Pro Ala Met Val Pro Pro Tyr Asp Leu
            660                 665                 670

Gly Met Ala Pro Asp Ala Ser Met Gln Leu Ser Ser Asp Met Gly Tyr
        675                 680                 685

Pro Pro Gln Ser Ile His Ser Phe Gln Ser Leu Glu Glu Ser Met Ser
    690                 695                 700

Val Leu Pro Ser Phe Gln Glu Pro His Leu Gln Met Pro Pro Asn Met
705                 710                 715                 720

Ser Gln Ile Thr Met Pro Phe Asp Gln Pro His Pro Gln Gly Leu Leu
                725                 730                 735

Gln Cys Gln Ser Gln Glu His Ala Val Ser Ser Pro Glu Pro Met Leu
            740                 745                 750

Cys Ser Asp Val Thr Met Val Glu Asp Ser Cys Leu Thr Gln Pro Val
        755                 760                 765

Gly Gly Phe Pro Gln Gly Thr Trp Val Ser Glu Asp Met Tyr Pro Pro
    770                 775                 780

Leu Met Pro Pro Thr Glu Gln Asp Leu Thr Lys Leu Leu Leu Glu Asn
785                 790                 795                 800

Gln Gly Glu Ala Gly Gly Ser Leu Gly Ser Gln Pro Leu Leu Gln Pro
                805                 810                 815

Ser Pro Tyr Gly Gln Ser Gly Ile Ser Leu Ser His Leu Asp Leu Arg
            820                 825                 830

Thr Asn Pro Ser Trp
        835

<210>  5
<211>  21
<212>  RNA
<213>  Artificial

<220>
<223>  Synthetic

<400>  5
gcuucugaua cguguaugag a                                                21

<210>  6
<211>  21
<212>  RNA
<213>  Artificial

<220>
<223>  Synthetic

<400>  6
uccgaagacu augcacauac u                                                21


<210>  7
<211>  21
<212>  RNA
<213>  Artificial

<220>
<223>  Synthetic

<400>  7
gauacgugua ugagacuaug c                                                21


<210>  8
<211>  21
<212>  RNA
<213>  Artificial

<220>
<223>  Synthetic

<400>  8
gacuaugcac auacucugau a                                                21


<210>  9
<211>  21
<212>  RNA
<213>  Artificial

<220>
<223>  Synthetic

<400>  9
gccgaggcac ccuguauauc c                                                21


<210>  10
<211>  21
<212>  RNA
<213>  Artificial

<220>
<223>  Synthetic

<400>  10
gacggcuccg ugggacauau a                                                21


<210>  11
<211>  21
<212>  RNA
<213>  Artificial

<220>
<223>  Synthetic

<400>  11
ccugguucug uuaaggauuc a                                                21

<210>  12
<211>  21
<212>  RNA
<213>  Artificial

<220>
<223>  Synthetic

<400>  12
ggggaccaag acaauuccua a                                                 21


<210>  13
<211>  21
<212>  RNA
<213>  Artificial

<220>
<223>  Synthetic

<400>  13
cgaaugugau acaacuguau c                                                 21


<210>  14
<211>  21
<212>  RNA
<213>  Artificial

<220>
<223>  Synthetic

<400>  14
gagcuuacac uauguugaca u                                                 21

## Description

### Technical Field

The present invention relates to a double-stranded RNA (dsRNA) molecule targeting the STAT6 gene and a pharmaceutical composition used for treatment or prevention of allergic diseases comprising such dsRNA molecule.

### Background Art

RNA interference (RNAi) is a phenomenon whereby mRNA is cleaved by double-stranded RNA (dsRNA) in a sequence-specific manner and gene expression is consequently suppressed. It is reported to be a defense mechanism at the nucleic acid level common among organisms (see Waterhouse, P. M. et al., Nature, 411: 834-843, 2001). Through RNAi, dsRNA is processed by the actions of a dicer, short interfering RNA (siRNA) is formed, siRNA functions as guide RNA for the recognition of a target sequence and the cleaving of target mRNA, and gene expression is thus suppressed.

A variety of examinations have been undertaken regarding RNAi-related technologies for the purpose of applying the same to gene function analysis via regulation of gene expression, elucidation of mechanisms of gene expression regulation, gene therapy, and the like.

Immune responses are classified into the cellular immunity mediated by the type-1 helper (Th1) cell and the humoral immunity mediated by the type-2 helper (Th2) cell. It is known that the Th1 immune responses are related to autoimmune diseases or graft rejection and the Th2 immune responses are related to allergic diseases. IL-4 and IL-13 are known as Th2-type cytokines related to Th2 immune responses.

It is reported that the STAT6 (signal transducers and activators of transcription 6) transcription regulators are important for signal transmission mediated by Th2-type cytokines (i.e., IL-4 and IL-13 receptors), and such regulators are associated with maintenance of Th2-type immune responses. It has been reported regarding STAT6 functions that STAT6-deficient mice were prepared, IL-4 information would not be transmitted to cells in such mice, and allergic reactions would not occur (see Takeda et al., Nature, Apr. 18, 1996, 380 (6575): 627-30; and Shimoda et al., Nature, Apr. 18, 1996; 380 (6575): 630-3).

### Disclosure of the Invention

It is an object of the present invention to provide double-stranded (ds) RNA capable of suppressing the expression of STAT6 related to allergic diseases via RNA interference mediated by Th2-type cytokines and a pharmaceutical composition for prevention or treatment of allergic diseases containing such dsRNA.

The present inventors have conducted concentrated studies in order to attain the above object. As a result, they discovered that a dsRNA molecule comprising a sense strand having a nucleotide sequence identical to the STAT6 gene sequence or a partial sequence thereof and an antisense strand having a nucleotide sequence complementary to a nucleotide sequence of the sense strand would specifically suppress STAT6 gene expression. Further, they discovered that suppression of STAT6 gene expression would result in suppression of Th2-type cytokine or chemokine production, and such suppression would be effective for prevention and treatment of allergic diseases. This has led to the completion of the present invention.

Specifically, the present invention is as follows.
[1] A double-stranded RNA (dsRNA) molecule targeting mRNA of the STAT6 gene composed of (a) or (b) below:
   (a) a sense strand identical to a sequence comprising 15 to 50 continuous nucleotides of the nucleotide sequence resulting from substitution of thymine with uracil in the STAT6 gene sequence represented by SEQ ID NO: 1 and an antisense strand comprising a nucleotide sequence complementary to the nucleotide sequence of the sense strand; or
   (b) a sense strand comprising a sequence derived from the sequence comprising 15 to 50 continuous nucleotides of the nucleotide sequence resulting from substitution of thymine with uracil in the STAT6 gene sequence represented by SEQ ID NO: 1 by deletion, substitution, or addition of 1 or several nucleotides and capable of hybridizing to the STAT6 gene and an antisense strand comprising a nucleotide sequence complementary to the nucleotide sequence of the sense strand.
[2] The dsRNA molecule according to [1] composed of (c) or (d) below:
   (c) a sense strand identical to a nucleotide sequence selected from the group consisting of: a sequence comprising nucleotides 3426 to 3446 of the nucleotide sequence resulting from substitution of thymine with uracil in the human STAT6 gene sequence represented by SEQ ID NO: 1; a sequence comprising nucleotides 3432 to 3452 of the nucleotide sequence resulting from substitution of thymine with uracil in the human STAT6 gene sequence represented by SEQ ID NO: 1 a sequence comprising nucleotides 259 to 279 of the nucleotide sequence resulting from substitution of thymine with uracil in the mouse STAT6 gene sequence represented by SEQ ID NO: 3; and a sequence comprising nucleotides 3026 to 3046 of the nucleotide sequence resulting from substitution of thymine with uracil in the mouse STAT6 gene sequence represented by SEQ ID NO: 3 and an antisense strand comprising a nucleotide sequence complementary to the nucleotide sequence of the sense strand; or
   (d) a sense strand comprising a sequence derived from any nucleotide sequence selected from the group consisting of: a sequence comprising nucleotides 3426 to 3446 of the nucleotide sequence resulting from substitution of thymine with uracil in the human STAT6 gene sequence represented by SEQ ID NO: 1; a sequence comprising nucleotides 3432 to 3452 of the nucleotide sequence resulting from substitution of thymine with uracil in the human STAT6 gene sequence represented by SEQ ID NO: 1; a sequence comprising nucleotides 259 to 279 of the nucleotide sequence resulting from substitution of thymine with uracil in the mouse STAT6 gene sequence represented by SEQ ID NO: 3; and a sequence comprising nucleotides 3026 to 3046 of the nucleotide sequence resulting from substitution of thymine with uracil in the mouse STAT6 gene sequence represented by SEQ ID NO: 3 by deletion, substitution, or addition of 1 or several nucleotides and capable of hybridizing to the STAT6 gene sequence and an antisense strand comprising a nucleotide sequence complementary to the nucleotide sequence of the sense strand.
[3] The dsRNA molecule according to [2], which is composed of any base pairs selected from the group consisting of: base pairs comprising a sense strand represented by SEQ ID NO: 5 and an antisense strand represented by SEQ ID NO: 6; base pairs comprising a sense strand represented by SEQ ID NO: 7 and an antisense strand represented by SEQ ID NO: 8; base pairs comprising a sense strand represented by SEQ ID NO: 9 and an antisense strand represented by SEQ ID NO: 10; base pairs comprising a sense strand represented by SEQ ID NO: 11 and an antisense strand represented by SEQ ID NO: 12; and base pairs comprising a sense strand represented by SEQ ID NO: 13 and an antisense strand represented by SEQ ID NO: 14.
[4] The dsRNA molecule according to any of [1] to [3], wherein the sense strand is ligated to the antisense strand via a linker molecule.
[5] A vector comprising template DNA of the dsRNA molecule according to [4] and expressing the dsRNA molecule.
[6] A pharmaceutical composition used for treatment or prevention of allergic diseases comprising 1 or a plurality of the dsRNA molecule(s) according to any of [1] to [4] and capable of suppressing STAT6 gene expression.
[7] A pharmaceutical composition used for treatment or prevention of allergic diseases comprising the vector according to [5] and capable of suppressing STAT6 gene expression.
[8] The dsRNA molecule according to any of [1] to [3], which has an overhanging nucleotide comprising one or a plurality of guanines (Gs) at the 5' end of the sense strand.
[9] The dsRNA molecule according to [8], wherein the sense strand is ligated to the antisense strand via a linker molecule.
[10] A vector comprising template DNA of the dsRNA molecule according to [9] and expressing the dsRNA molecule.
[11] A pharmaceutical composition used for treatment or prevention of allergic diseases comprising 1 or a plurality of the dsRNA molecule(s) according to [8] or [9], which induces no interferon and/or cell damage and is capable of suppressing STAT6 gene expression.
[12] A pharmaceutical composition used for treatment or prevention of allergic diseases comprising the vector according to [10], which induces no interferon and/or cell damage and is capable of suppressing STAT6 gene expression.
[13] The pharmaceutical composition used for treatment or prevention of allergic diseases according to [6], [7], [11], or [12], which is administered through the nasal cavity.
[14] The pharmaceutical composition used for treatment or prevention of allergic diseases according to [6], [7], [11], or [12], which is an ointment to be applied to the skin.

This description includes part or all of the contents as disclosed in the description and/or drawings of Japanese Patent Application No. 2008-237007, which is a priority document of the present application.

### Brief Description of the Drawings

Fig. 1 is a schematic diagram showing the experimental protocols 1 (A) and 2 (B) of the examples.
Fig. 2 shows the structure of siRNA and that of shRNA.
Fig. 3 shows the suppression of STAT6 expression via introduction of human STAT6 siRNA.
Fig. 4 shows eotaxin-3 production by human fibroblasts into which human STAT6 siRNA has been introduced.
Fig. 5 shows the suppression of STAT6 expression at the mRNA level in human fibroblasts into which human STAT6 shRNA has been introduced.
Fig. 6 shows the suppression of STAT6 expression at the protein level in human fibroblasts into which human STAT6 shRNA has been introduced.
Fig. 7 shows eotaxin-3 production by human fibroblasts into which human STAT6 shRNA has been introduced.
Fig. 8 shows the results of the suppression of STAT6 expression in normal fibroblasts via introduction of mouse STAT6 siRNA, confirmed via Western blotting.
Fig. 9 shows the results of the suppression of STAT6 expression in normal fibroblasts via introduction of mouse STAT6 siRNA, confirmed via RT-PCR.
Fig. 10 shows the results of quantification via ELISA of the concentration of eotaxin (CCL11) produced by IL-4 and TNF-alpha costimulation in normal fibroblasts via introduction of mouse STAT6 siRNA.
Fig. 11 shows the effects of STAT6 siRNA on contact hypersensitivity responses. Fig. 11A shows the effects of TNCB on contact hypersensitivity responses, Fig. 11B shows the effects of DNFB on contact hypersensitivity responses, and Fig. 11C shows the effects of oxazolone on contact hypersensitivity responses.
Fig. 12 shows the effects of STAT6 siRNA on contact hypersensitivity responses via Giemsa staining.
Fig. 13 shows the effects of STAT6 siRNA on contact hypersensitivity responses using profiles of infiltrated cells.
Fig. 14 shows the effects of a STAT6 siRNA-containing ointment on contact hypersensitivity responses.
Fig. 15 shows the effects of STAT6 siRNA on rhinitis models based on the number of times of sneezing.
Fig. 16 shows the effects of STAT6 siRNA on rhinitis models via Giemsa staining.
Fig. 17 shows the effects of STAT6 siRNA on rhinitis models based on the number of infiltrated eosinophils.

### Best Modes for Carrying out the Invention

Hereafter, the present invention is described in detail.

The present invention relates to a double-stranded RNA (dsRNA) molecule targeting mRNA of the STAT6 (signal transducers and activators of transcription 6) transcription regulator. A strand of the dsRNA molecule is a sense strand having a nucleotide sequence identical to the gene sequence of the STAT6 transcriptional factor or a partial sequence thereof and capable of hybridizing thereto. Another strand is an antisense strand comprising a nucleotide sequence complementary to the nucleotide sequence of the sense strand. The sense strand complementarily binds to the antisense strand. In such a case, it is not necessary that the sense strand and the antisense strand are completely complementary to each other. As long as such sequences complementarily bind, 1 or a plurality of mismatches; i.e., 1 to 10, preferably 1 to 5, more preferably 1 to 3, and 2 or 1 mismatches may be present. A target sequence in the STAT6 gene may be a coding or non-coding region.

In the present invention, the term "dsRNA molecule" refers to the siRNA molecule and the shRNA molecule. In the present invention, the dsRNA molecule is capable of forming the miRNA molecule.

The number of nucleotides in the target sequence of the STAT6 gene of the dsRNA molecule of the present invention is not particularly limited. It is 15 to 50, 15 to 45, 15 to 40, 15 to 35, or 15 to 30 nucleotides, preferably 20 to 35 nucleotides, more preferably 21 to 35, 21 to 25, or 21 to 23 nucleotides, and particularly preferably 21 nucleotides.

In the present invention, the term "a sense strand having a nucleotide sequence identical to the STAT6 gene sequence or a partial sequence thereof and capable of hybridizing thereto" refers to an RNA sequence having the nucleotide sequence resulting from substitution of thymine with uracil in the human STAT6 gene sequence represented by SEQ ID NO: 1 or the mouse STAT6 gene sequence represented by SEQ ID NO: 3. A sense strand constituting the dsRNA molecule of the present invention is preferably identical to the STAT6 gene sequence, and a substantially identical sequence may be sufficient. As long as the sense strand of the dsRNA molecule hybridizes to the actual target STAT6 mRNA sequence, specifically, a mismatch may occur via deletion, substitution, or addition of 1 or a plurality of nucleotides (i.e., 1 to 10, preferably 1 to 5, more preferably 1 to 3, or 2 or 1 nucleotides). In such a case, hybridization is carried out under *in vivo* conditions when the dsRNA of the present invention is used in the form of a pharmaceutical agent via administration thereof to an organism. When dsRNA of the present invention is used in the form of a reagent *in vitro,* hybridization is carried out under moderately to highly stringent conditions. An example thereof is a condition in which hybridization is carried out in the presence of 400 mM NaCl, 40 mM PIPES (pH: 6.4), and 1 mM EDTA at 50°C to 70°C for 12 to 16 hours.

The sense strand of the dsRNA of the present invention has 90% or higher, preferably 95% or higher, and more preferably 96, 97, 98, or 99% or higher sequence identity to the target sequence, which is determined with the use of default parameters (i.e., the initially set parameters) via a homology search program known to a person skilled in the art, such as BLAST (J. Mol. Biol., 215, 403-410, 1990) or FASTA (Methods Enzymol., 183, 63-98, 1990).

The dsRNA molecule of the present invention can be provided in the form of a short hairpin RNA (shRNA) molecule in which the sense strand and the antisense strand defined above are ligated to each other via a linker molecule, and a loop structure is formed and folded at the linker site. The shRNA molecule is processed by a dicer and it is capable of forming a siRNA molecule. As long as a linker molecule contained in the shRNA molecule is capable of ligating a sense strand to an antisense strand to form a stem-loop structure, a polynucleotide linker or non-polynucleotide linker may be used. A polynucleotide linker known to a person skilled in the art is preferable, although it is not limited thereto. A hairpin-loop sequence is not limited. An example thereof is a sequence comprising 5 to 12 nucleotides and beginning with UU (e.g., UUCAAGAGA). Loop sequences described in, for example, Lee NS. et al., 2002, Nat. Biotech., 20, 500-505, Paddison P. J. et al., 2002, Genes and Dev. 16, 948-958, Sui G. et al., 2002, Proc. Natl. Acad. Sci. USA 99, 5515-5520, Paul CP. et al., 2002, Nat. Biotech. 20, 505-508, and Kawasaki H. et al., 2003, Nucleic Acids Res. 31, 700-707, can be adopted. The shRNA molecule can be synthesized *in vitro* or *in vivo* in accordance with known techniques as described above. When synthesizing the shRNA molecule, a single RNA strand comprising a sense strand and an inverted antisense strand is synthesized, and the single RNA strand is converted into a double-strand via self-complementary binding. Thus, the shRNA molecule can be obtained.

The sense or antisense strand constituting the dsRNA molecule may comprise an overhang at the 3' end according to need. The type and the number of nucleotides constituting such overhang are not limited. For example, a sequence comprises 1 to 5, preferably 1 to 3, and more preferably 1 or 2 nucleotides. Examples of such sequence include TTT, UU, and TT. In the present invention, the term "overhang" refers to a nucleotide added to the end of a strand of the dsRNA molecule that does not have a nucleotide at a corresponding site of another strand to which the overhang can complementarily bind. An overhang may comprise nucleotides that naturally constitute DNA of the target STAT6 gene. For example, the dsRNA of the present invention is composed of a sense strand comprising 21 continuous nucleotides in the nucleotide sequence resulting from substitution of thymine with uracil in the STAT6 gene sequence, an antisense strand having a sequence complementary to a nucleotide sequence lacking 2 nucleotides at the 3' end of the sense strand, and an overhang of two nucleotides at the 3' end of the sense or antisense strand.

Also, the dsRNA of the present invention may have at the 5' end of the sense strand an overhang comprising 1 to 3, and preferably 3, 2, or 1 guanine(s) (Gs). By providing an overhang comprising 1 or a plurality of Gs at the 5' end of the sense strand, interferon expression would not be induced in cells into which shRNA had been introduced (Gondai et al., Nucleic Acids Research, 2008, Vol. 36, No. 3, e18, Epub., Jan. 21, 2008).

In addition, the sense or antisense strand constituting the dsRNA molecule may comprise substitution, addition, or deletion of 1 to 3 nucleotides, and preferably 1 or 2 nucleotides, according to need, in order to smoothly carry out various experimental operations, such as gene sequencing, provided that siRNA would not be influenced.

The 5' end of the sense or antisense strand may be phosphorylated according to need. Triphosphoric acid (ppp) may bind to the 5' end of the shRNA molecule. Triphosphoric acid (ppp) may bind to the overhang comprising G at the 5' end of the sense strand as described above.

In the present invention, a sense strand is preferably identical to a nucleotide sequence selected from the group consisting of: a sequence comprising nucleotides 3426 to 3446 of the nucleotide sequence resulting from substitution of thymine with uracil in the human STAT6 gene sequence represented by SEQ ID NO: 1; a sequence comprising nucleotides 3432 to 3452 of the nucleotide sequence resulting from substitution of thymine with uracil in the human STAT6 gene sequence represented by SEQ ID NO: 1; a sequence comprising nucleotides 259 to 279 of the nucleotide sequence resulting from substitution of thymine with uracil in the mouse STAT6 gene sequence represented by SEQ ID NO: 3; and a sequence comprising nucleotides 3026 to 3046 of the nucleotide sequence resulting from substitution of thymine with uracil in the mouse STAT6 gene sequence represented by SEQ ID NO: 3. It is particularly preferable that a sense strand be identical to a sequence comprising nucleotides 3426 to 3446 of the nucleotide sequence resulting from substitution of thymine with uracil in the human STAT6 gene sequence represented by SEQ ID NO: 1 or a sequence having nucleotides 3432 to 3452 of the nucleotide sequence resulting from substitution of thymine with uracil in the human STAT6 gene sequence represented by SEQ ID NO: 1.

The dsRNA molecule of the present invention comprises base pairs selected from the group consisting of: base pairs comprising a sense strand represented by SEQ ID NO: 5 and an antisense strand represented by SEQ ID NO: 6; base pairs comprising a sense strand represented by SEQ ID NO: 7 and an antisense strand represented by SEQ ID NO: 8; base pairs comprising a sense strand represented by SEQ ID NO: 9 and an antisense strand represented by SEQ ID NO: 10; base pairs comprising a sense strand represented by SEQ ID NO: 11 and an antisense strand represented by SEQ ID NO: 12; and base pairs comprising a sense strand represented by SEQ ID NO: 13 and an antisense strand represented by SEQ ID NO: 14. A dsRNA molecule composed of a sense strand represented by SEQ ID NO: 5 and an antisense strand represented by SEQ ID NO: 6 or a sense strand represented by SEQ ID NO: 7 and an antisense strand represented by SEQ ID NO: 8 is particularly preferable.

The dsRNA of the present invention can be chemically synthesized or synthesized *in vitro* via a transcription system using a promoter and RNA polymerase. In the case of chemical synthesis, single-stranded RNA having inverted complementary sequences and having self-complementarity may be synthesized, and such sequences may be bound to each other at the self-complementarity region. The synthesized sense and antisense strands can be annealed by a common method known to a person skilled in the art. The synthesized sense and antisense strands are dissolved in a buffer for dsRNA annealing, equal amounts (equimolar numbers) thereof are mixed, temperature is raised until double strands are dissociated from each other, and the resultants are incubated via gradual cooling. Thus, dsRNA annealing can be carried out. Annealing may be carried out by allowing the dsRNA to stand at 90°C for 1 minute and then at 37°C for 1 hour, for example. Thereafter, phenol/chloroform extraction and ethanol precipitation may be carried out to obtain the dsRNA molecule. Synthesis using a promoter and RNA polymerase may be carried out by synthesizing template DNA having a promoter and, at a site downstream thereof, a sense strand ligated to an antisense strand to form a loop structure and transcribing RNA with the aid of RNA polymerase. In order to add an overhang sequence of G to the 5' end of the sense strand, a sequence comprising G may be added to the end of a promoter. In this case, a DNA sequence adequately comprises a regulator sequence, such as a terminator. Promoter and/or other regulator sequences are functionally linked to a vector. The term "functionally linked" used herein refers to a situation in which promoter and/or other regulator sequences are linked and incorporated into a vector, so that the dsRNA molecule is expressed and target STAT6 mRNA is degraded in cells into which the vector has been introduced under the control of the promoter and/or other regulator sequences. As a terminator, for example, a TTTTTT sequence may be used. As a promoter, a constitutive promoter, a tissue-specific promoter, a stage-specific promoter, or the like can be used. In the case of *in vitro* production, T3 promoter, T7 promoter, or the like may be used. When template DNA of double-stranded RNA of the present invention is introduced into a vector and such vector is administered to an organism to synthesize double-stranded RNA *in vivo,* PolIII promoters, such as U6 promoter and H1 promoter, are used. When a vector is used, a plasmid, virus, or other vector can be used. pBAsi, pSUPER, and other vectors may be used as plasmid vectors, and adenovirus, lentivirus, retrovirus, and other vectors may be used as virus vectors. When T7 promoter or the like is used for synthesizing the dsRNA of the present invention, the existence of a sequence of G activates T7 promoter, and the efficiency for dsRNA production is advantageously enhanced.

The present invention also includes a vector that expresses the dsRNA molecule described above.

The dsRNA molecule of the present invention is capable of cleaving STAT6 mRNA in a sequence-specific manner, inducing RNA interference (RNAi) that suppresses STAT6 gene expression, and consequently knocking down the STAT6 gene. As a result of STAT6 gene knockdown, production of Th2 cytokine and chemokine is suppressed. For example, infiltration of mononuclear cells, eosinophils, neutrophils, mast cells, lymphocytes, and the like is suppressed at inflammatory sites, such as skins, and inflammation is suppressed.

When the dsRNA molecule of the present invention is provided in the length of 30 nucleotides or longer, it is processed by the action of an RNaseIII-like enzyme that is referred to as a dicer, and a small interfering RNA (siRNA) molecule comprising 21 to 27 nucleotides having an overhang of 2 nucleotides at the 3' end can be formed. Such siRNA molecule is incorporated into a protein complex referred to as the RNA-induced silencing complex (RISC), and it recognizes and degrades STAT6 mRNA based on its homology to the siRNA molecule to suppress STAT6 gene expression.

The dsRNA molecule of the present invention can be used for treatment and prevention of allergic diseases. Examples of allergic diseases include rhinostenosis, allergic bronchitis, allergic conjunctivitis, inflammatory disease, rash, hives, atopic dermatitis, allergic rhinitis (pollinosis), allergic conjunctivitis, allergic gastroenteritis, bronchial asthma, pediatric asthma, alimentary allergy, drug allergy, and inflammatory diseases.

The present invention includes a pharmaceutical composition used for treatment or prevention of allergic diseases comprising the dsRNA molecule targeting the STAT6 gene. Such composition may comprise 1 or a plurality of types of the dsRNA molecules of the present invention or vectors in combination. For example, the composition may comprise 1, 2, or 3 types of dsRNA molecules selected from the group consisting of: a dsRNA molecule comprising a sense strand represented by SEQ ID NO: 5 and an antisense strand represented by SEQ ID NO: 6; a dsRNA molecule comprising a sense strand represented by SEQ ID NO: 7 and an antisense strand represented by SEQ ID NO: 8; a dsRNA molecule comprising a sense strand represented by SEQ ID NO: 9 and an antisense strand represented by SEQ ID NO: 10; a dsRNA molecule comprising a sense strand represented by SEQ ID NO: 11 and an antisense strand represented by SEQ ID NO: 12; and a dsRNA molecule comprising a sense strand represented by SEQ ID NO: 13 and an antisense strand represented by SEQ ID NO: 14.

When analytes are cells or tissue, the dsRNA of the present invention can be introduced by culturing the dsRNA simultaneously with such cells or tissue. Also, a method involving the use of calcium ions, electroporation, the spheroplast method, the lithium acetate method, the calcium phosphate method, lipofection, microinjection, or other methods may be employed to introduce dsRNA. When an analyte is an individual animal, dsRNA can be administered through an oral route, a nasal route, intravenous, intramuscular, subcutaneous, or intraperitoneal injection, or a non-enteral route. In order to treat asthma, rhinitis, or other diseases, dsRNA can be administered in the form of a liquid or aerosol mixture with a known penetrating agent. In order to treat dermatitis or the like, it can be administered topically by applying the same in the form of an ointment to the skin lesion. Such ointment comprises, as a carrier, fat, fatty oil, lanolin, petroleum jelly, paraffin, wax, plaster, resin, plastic, glycols, higher alcohol, glycerin, water or an emulsifier, or a suspending agent. In such a case, dsRNA may be administered in the form of a mixture with a positively charged cationic polymer. Examples of cationic polymers include polyethyleneimine (PEI: linear or branched), polyamine, and commercially available gene transfer reagents. Examples of commercially available gene transfer reagents include Lipofectamine®, Lipofectamine 2000, and Lipofectamine RNAiMAX.

When dsRNA is administered to a specific site, it may be delivered to such site with the utilization of a drug delivery system. A variety of known drug delivery systems are available, and an adequate means can be adopted in accordance with a site of interest. Examples of drug delivery systems include known methods utilizing liposomes, emulsions, and polylactic acids as carriers. It is preferable that dsRNA be administered in the form of a mixture with a pharmaceutically acceptable diluent or carrier. Examples of adequate carriers include, but are not limited to, physiological saline, phosphate buffered saline, phosphate buffered saline in a glucose solution, and buffered saline. The amount of dsRNA to be introduced can be adequately determined in accordance with, for example, a type of disease to be prevented or treated, severity thereof, and age, body weight, and other conditions of a subject. It is preferable that at least a copy of dsRNA be introduced per cell in a lesion area. For example, 1 nM to 100 µM, preferably 10 nM to 50 µM, and more preferably 100 nM to 20 µM dsRNA molecules are administered per dose.

According to the present invention, the condition in which STAT6 gene expression is suppressed (silenced) via RNA interference refers to a situation in which such gene expression is suppressed by 75% or more, 50% or more, or 20% or more, as well as 100%, when the STAT6 gene expression level is determined using the gene expression level at the mRNA or protein level as the indicator, compared with the case in which the dsRNA of the present invention is not introduced. The degree of expression suppression may be determined by comparing the amounts of mRNA or protein production of the STAT6 gene before and after the introduction of dsRNA. In the case of mRNA, the degree of expression suppression can be determined via Northern hybridization, RT-PCR, *in situ* hybridization, or other means. In the case of proteins, such degree can be determined via Western blotting, ELISA, assay involving the use of a protein chip to which antibodies have been bound, protein activity assay, or other means. The dsRNA of the present invention having at the 5' end of the sense strand an overhang comprising 1 or several Gs would not induce interferon reactions in the cells or organisms into which such dsRNA has been introduced. The situation in which it would not induce interferon reactions refers to a situation in which interferon α or β expression is not induced. It also refers to not only a situation in which interferon synthesis would not be activated but also a situation in which a pathway associated with interferon would not be activated. A case in which interferon expression is suppressed by 75% or more, 50% or more, 20% or more, or 10% or more is within the scope of the present invention as well as a case in which it is suppressed completely. Whether or not the interferon reaction has occurred can be determined by assaying the production of interferon or interferon mRNA. Such assay may be carried out via Northern hybridization, RT-PCR, *in situ* hybridization, Western blotting, ELISA, assay involving the use of a protein chip to which antibodies have been bound, protein activity assay, or other means as described above.

The dsRNA of the present invention would not cause cell damage to cells or cells of an organism even if it is introduced thereinto. When double-stranded RNA is administered, in general, cell damage would be induced via activation of dsRNA-dependent protein kinase (PKR). However, the dsRNA of the present invention would not activate PKR or cause cell damage. The term "cell damage" used herein refers to a situation in which cells are damaged to the extent that normal functions are not exerted or growth is suppressed. The term also refers to a cell death, such as apoptosis or necrosis. In the present invention, the term "...would not cause cell damage" refers not only a situation in which cell damage would not be induced at all but also a situation in which the number of cells experiencing cell damage is 75% or less, 50% or less, 20% or less, or 10% or less than the number of such cells when double-stranded RNA comprising a sense strand without a sequence of G at the 5' end is introduced. Whether or not cell damage is induced can be determined by observing cells and inspecting the development of the cytopathic effects (CPE). Also, it can be determined by assaying the metabolic activity of cells or a dye-exclusion test, such as trypan blue-exclusion. The dsRNA of the present invention would not induce both or either of interferon and cell damage. In the present invention, the situation in which "inducing no interferon and/or cell damage" refers to not only a situation in which induction is completely inhibited but also a situation in which induction is reduced. The degree of inhibition of interferon and/or cell damage induction may vary depending on a test system, an analyte, and other conditions. dsRNA that does not induce interferon and/or cell damage in at least 1 system or analyte is within the scope of the dsRNA of the present invention that does not induce interferon and/or cell damage.

Further, the present invention includes a method of administering the dsRNA of the present invention to an organism and suppressing STAT6 gene expression in the organism without inducing interferon expression and a method of administering the dsRNA of the present invention to an animal in order to prevent or treat allergic diseases associated with the STAT6 gene without inducing interferon expression.

The present invention is described in detail with reference to the following examples, although the present invention is not limited to these examples.

### Method

### Animal

BALB/c mice were purchased from Sankyo Labo Service Corporation. All mice were raised by supplying feeds and water at the sterile facilities in accordance with the Guidelines for Animal Experimentation of Tokyo Medical and Dental University. 7- to 12-week-old mice were subjected to the experiment, and a group consisted of at least 4 mice.

### Preparation of human STAT6 small interfering RNA (siRNA) and small hairpin RNA (shRNA) and evaluation of effects thereof

Small RNAs to be subjected to the experiments (i.e., STAT6 siRNA and STAT6 shRNA) were prepared by Tokyo Medical and Dental University in collaboration with HaploPharma Inc. Based on the theory leading to more potent RNAi than existing RNAi (Ui-Tei K, et al., Nucleic Acids Res., 2004, 32: 936-948), siRNA candidate sequences were first determined by using the BLAST Search (http://www.ncbi.nml.nih,gov/blast/index.shtml; avoidance of off-target effects) and predicting the mRNA higher-order structure (http://www.bioinfo.rpi.edu/~zukerm/).

Six candidate sequences were selected, and the effects thereof were evaluated by introducing such sequences into normal human dermal fibroblasts sampled from human skin tissue biopsy specimens. After siRNA of the sequence exhibiting efficient RNA interference was selected, shRNA exhibiting substantially no non-specific reactions (interferon responses) at the time of introduction was prepared based on such sequence, and the RNAi effects thereof were also examined.

### Selection of mouse STAT6 siRNA candidate

Since use of mice was planned for the *in vivo* experiment, it was necessary to examine mouse STAT6 siRNA *in vitro* as in the case of human STAT6 siRNA. Based on the theory same as that for human STAT6 siRNA, 3 types of candidate sequences for mouse STAT6 siRNA were designed. The effects of RNA interference were evaluated using normal fibroblasts sampled from the dorsal skin of newborn mice.

### Cell culture and stimulation

STAT6 siRNA was introduced into cells using Lipofectamine™ 2000 (Invitrogen).

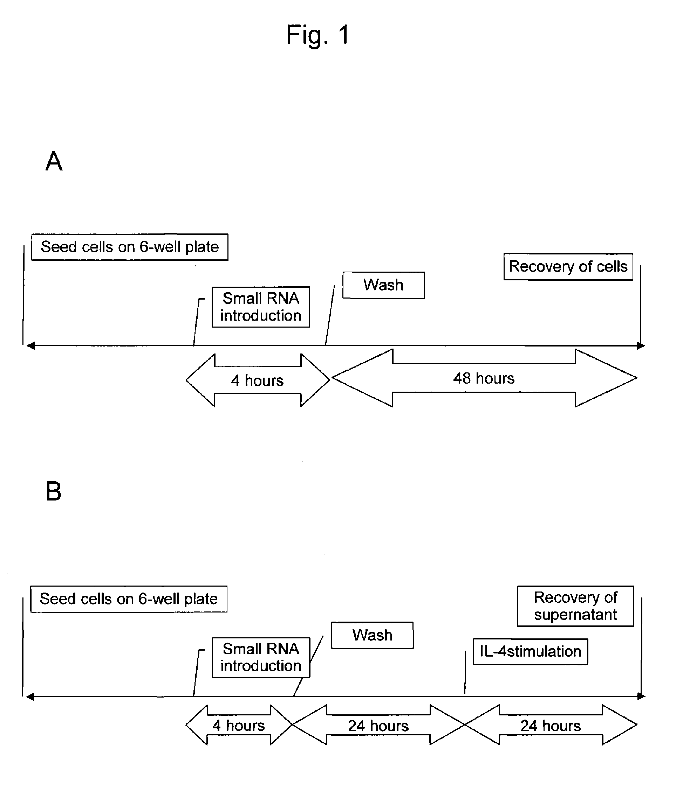

STAT6 siRNA was introduced into cells based on two types of protocols. The protocols are shown in Fig. 1A and Fig. 1B.

### Experimental protocol 1

Normal fibroblasts are seeded on a 6-well plate. Culture is conducted using a culture solution prepared by adding 10% fetal calf serum (FCS, Sigma) and 1% antibiotics/antimycotics (Gibco-BRL) to the Dullbeco's modified Eagle's medium (DMEM, Sigma) at 37°C in the presence of 5% CO₂. siRNA or shRNA is introduced under 60% to 70% subconfluent conditions, culture is continued for an additional 48 hours, and cells are then recovered.

### Experimental protocol 2

Introduction of siRNA or shRNA is carried out in the same manner as in Protocol 1. After siRNA or shRNA is introduced, the culture solution is replaced with another culture solution prepared by adding 2% FCS and 1% antibiotics/antimycotics to DMEM (serum starvation). Cells were stimulated with rhIL-4 (with 10 ng/ml rmIL-4 and 40 ng/ml TNFα in the case of mouse, R & D) 24 hours later, and the cell culture supernatant is recovered 24 hours thereafter.

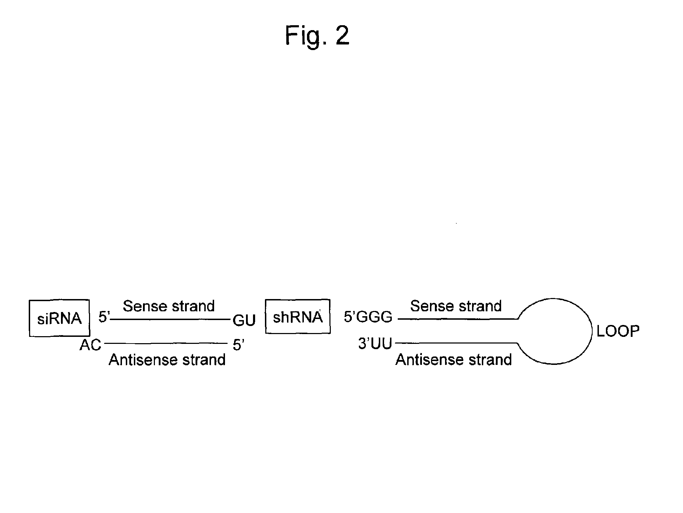

Since shRNA is introduced into cells before it is degraded by a dicer, shRNA functions more specifically than siRNA that separately expresses double strands, and it is capable of reducing the interferon responses caused by introduction. Fig. 2 shows the structure of shRNA and that of siRNA.

### Induction of acute contact hypersensitivity responses by hapten

Mice were sensitized by applying 50 µl of 5% 2,4,6-trinitrochlorobenzene (TNCB) (in acetone:olive oil = 4:1) (Nacalai Tesque), 0.5% 2,4-dinitrofluorobenzene (DNFB) (in acetone: olive oil = 4:1) (Nacalai Tesque), or 5% 4-ethoxymethylene-2-phenyl-2-oxazolin-5-one (oxazolone) (in acetone: olive oil = 4:1) (Sigma) to the shaved abdominal region on Day 0 (DNFB was applied continuously on Days 0 and 1). Thereafter, 20 µl of 1% TNCB (in acetone:olive oil = 1:4), 0.2% DNFB (in acetone:olive oil = 4:1), or 1% oxazolone (in acetone:olive oil = 4:1) was applied to the auricles on Day 5 to elicit the response. Auricular swelling was measured using a dial thickness gauge (Peacock) as the indicator for the contact hypersensitivity responses.

### Western blotting

Proteins were extracted from cells using a cell lysis buffer (Cell Signaling). After electrophoresis was carried out, the product was transferred to Hybond-P (Amersham Pharmacia Biotech). Electrophoresis was carried out at 80 V for 30 minutes and then at 130 V for 60 minutes. The reaction was allowed to proceed with the use of anti-human β-actin antibodies or anti-mouse β-actin antibodies and anti-human STAT6 antibodies or anti-mouse STAT6 antibodies (Santa Cruz Biotechnology) as the primary antibodies, and the reaction was then carried out with the use of peroxidase-labeled anti-rabbit immunoglobulins antibodies (DAKO) as the secondary antibodies. Color development was carried out using the ECL plus Western blotting detection reagents (GE Healthcare).

### RNA extraction and reverse transcription

RNA was extracted from normal fibroblasts using ISOGEN (Nippon Gene Co., Ltd.). Reverse transcription was carried out using a reaction buffer containing a hexanucleotide mixture (A260, 6.25 U/ml, Roche), dNTPs (0.125 mM, Takara Bio), the human placenta RNase inhibitor (80 U, Takara Bio), and a reverse transcriptase (400 U, Moloney murine leukemia virus, Takara Bio) and 800 ng of RNA. The total amount of the reaction solution was adjusted to 40 µl, and the reaction was allowed to proceed at 37°C for 60 minutes.

### Quantitative polymerase chain reaction (PCR)

Quantitative PCR was carried out using the Brilliant SYBR Green QPCR Master Mix (Stratagene) in accordance with the protocols. Measurement was carried out using the Mx3000P Real-Time PCR system (Stratagene).

### Administration of STAT6 siRNA to mice

On Day 3, 30 µl of STAT6 siRNA (in OPTI-MEM® I:Lipofectamine™2000 = 50:1) was administered subcutaneously to the auricles (1 nmol/ear). STAT6 siRNA-containing ointment

The STAT6 siRNA-containing ointment was prepared using hydrophilic petrolatum as a base to adjust the concentration of STAT6 siRNA to 2%. The resulting ointment was applied to the auricles on Day 3 in an amount of 10 nmol/ear.

### Preparation of rhinitis mouse models

Mice were sensitized via intraperitoneal administration of 0.1 mg of ovalbumin (albumin from chicken egg white, Grade V, OVA, Sigma) and 1 mg of alum on Days 0, 7, 14, and 21. OVA was administered through the nasal cavity by inhalation in an amount of 0.2 mg/day successively from Days 21 to 27 to elicit symptoms. As the indicator for rhinitis symptoms, the number of times of sneezing for 5 minutes was counted immediately after the final elicitation on Day 27.

### Histopathological examination

Auricle tissue samples were recovered from the models for contact hypersensitivity responses and fixed in 10% formalin to prepare paraffin blocks. The resultants were sliced and histopathologically examined via May Grunwald/Giemsa staining. Tissue slices obtained from the nasal mucous membrane of rhinitis models 12 hours after the final elicitation were histopathologically examined via May Grunwald/Giemsa staining. The infiltrated mononuclear cells, neutrophils, eosinophils, and mast cells were counted at 400-fold magnification in at least 5 fields and quantified.

### Statistical analysis

A statistically significant difference was examined by the student's t-test. Results

### 1. Suppression of STAT6 expression by introduction of human STAT6 siRNA

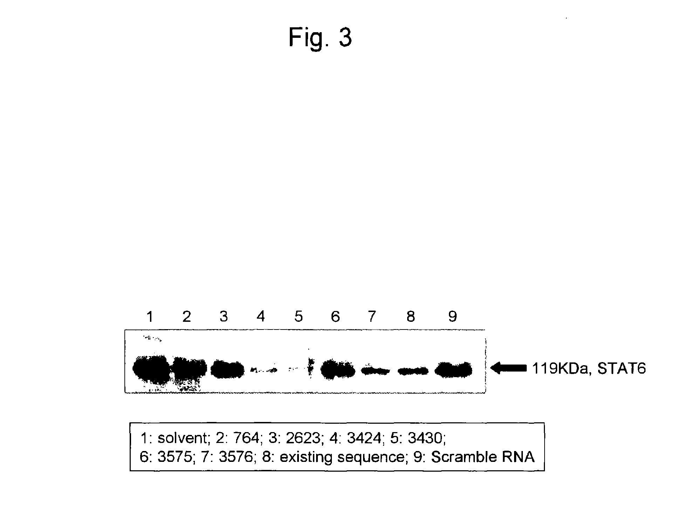

The 6 prepared candidate sequences for human STAT6 siRNA were subjected to Western blotting so as to determine a sequence capable of efficiently suppressing STAT6 expression. The results are shown in Fig. 3. In Fig. 3, lanes 2 to 7 represent the 6 types of STAT6 siRNAs that were newly prepared this time, and lane 8 represents an existing sequence (Rippmann J. F. et al., FEBS Lett., 2005, 579: 173-178). Among the newly prepared STAT6 siRNAs, those represented by lanes 4 and 5 show the effects of STAT6 expression suppression that are more potent than those attained by existing STAT6 siRNAs at a significant level.

Therefore, STAT6 siRNAs having such 2 sequences were to be used for the following experiment (hereafter, "3424" represents STAT6 siRNA 3 and "3430" represents STAT6 siRNA 4). The nucleotide sequences are as shown below.
3424 (STAT6 siRNA 3)
5'-GCUUCUGAUACGUGUAUGAGA sense strand (SEQ ID NO: 5)
UCCGAAGACUAUGCACAUACU-5' anti-sense strand (SEQ ID NO: 6)
3430 (STAT6 siRNA 4)
5'-GAUACGUGUAUGAGACUAUGC sense strand (SEQ ID NO: 7)
GACUAUGCACAUACUCUGAUA-5' anti-sense strand (SEQ ID NO: 8)

### 2. Production of eotaxin-3 by human fibroblast into which human STAT6 siRNA has been introduced

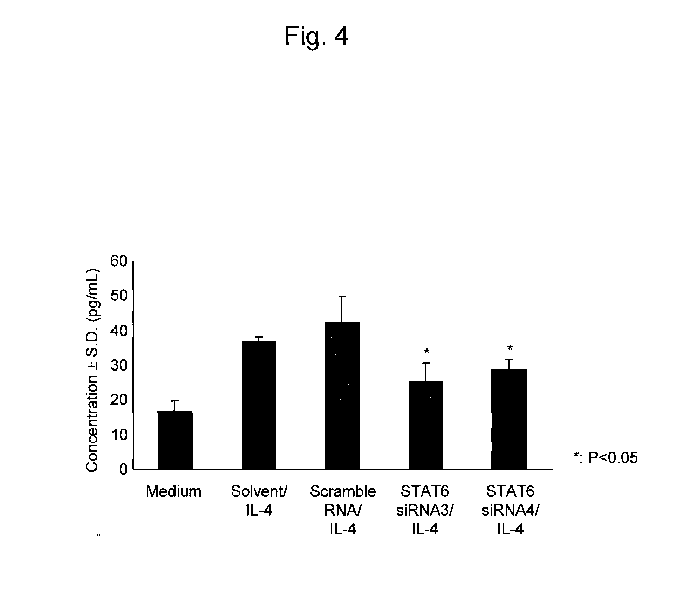

Upon IL-4 stimulation, production of eotaxin-3 by human fibroblasts depending on the STAT6 pathway was significantly suppressed via introduction of STAT6 siRNA 3 or STAT6 siRNA 4 selected in Experiment 1. Fig. 4 shows the results of ELISA assay. Without stimulation, production of eotaxin-3 from fibroblasts was not influenced (data not shown).

### 3. Suppression of STAT6 expression in human fibroblasts into which human STAT6 shRNA had been introduced

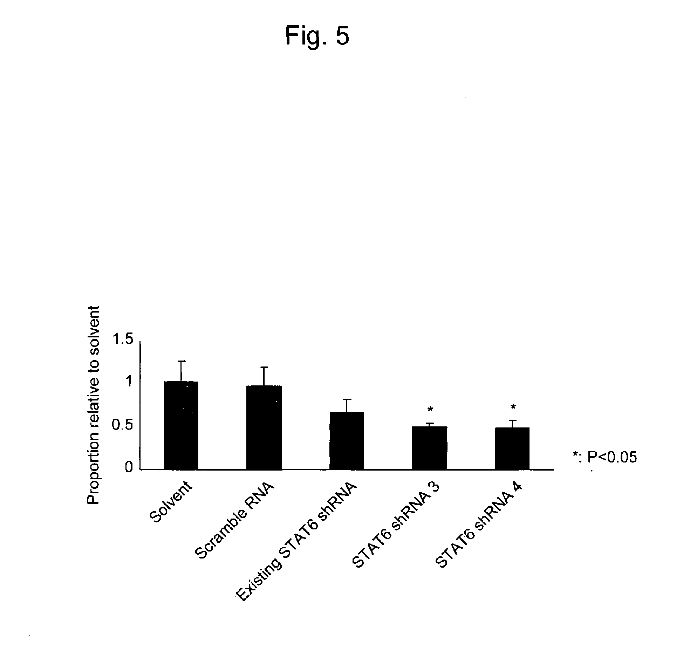

shRNAs were prepared based on the 2 siRNA sequences that had exhibited the effects in Experiments 1 and 2 (STAT6 shRNA3 and STAT6 shRNA4). The effects thereof on STAT6 expression *in vitro* were examined. As with the results regarding siRNA, two new sequences were found to produce more potent effects of suppressing STAT6 expression at the mRNA level via real-time PCR. Fig. 5 shows the results of ELISA assay.

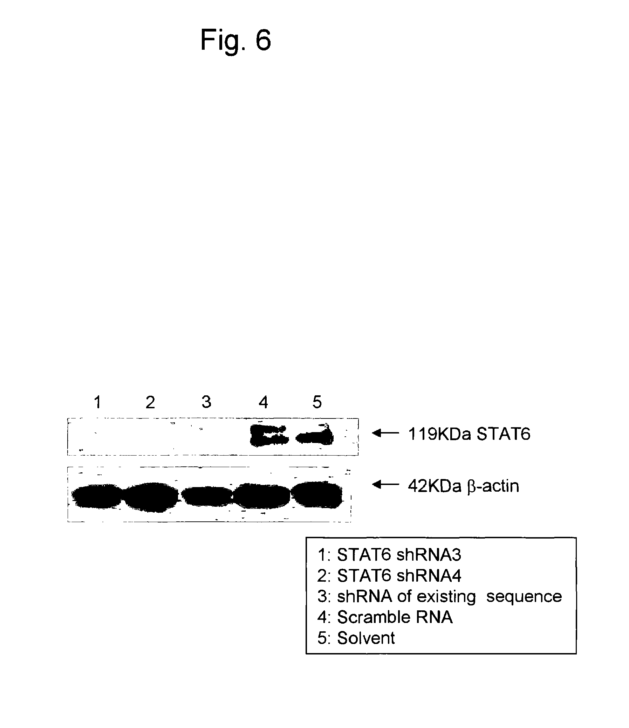

STAT6 expression at the protein level examined via Western blotting was found to be suppressed upon shRNA introduction. While shRNA prepared based on existing sequences shows the equivalent effects of suppressing STAT6 expression (lane 3 in Fig. 6), the newly prepared sequences are considered to have more potent suppression effects, in comparison with β-actin (Fig. 6).

### 4. Production of eotaxin-3 in human fibroblasts into which human STAT6 shRNA had been introduced

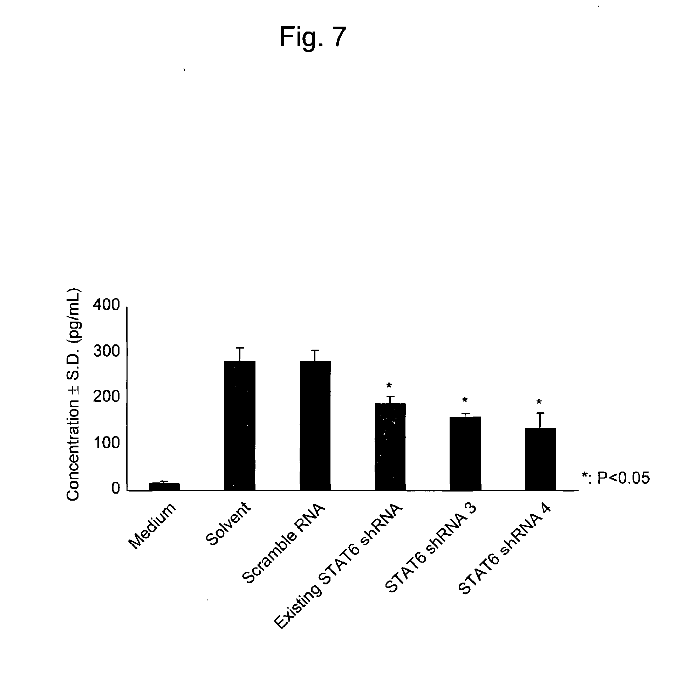

As with the case in which siRNA had been introduced, the newly prepared sequence exhibited more potent effects of suppressing eotaxin-3 expression than existing sequences upon shRNA introduction. Fig. 7 shows the results of ELISA assay.

### 5. Effects of introduction of mouse STAT6 siRNA on STAT6 expression in normal fibroblasts

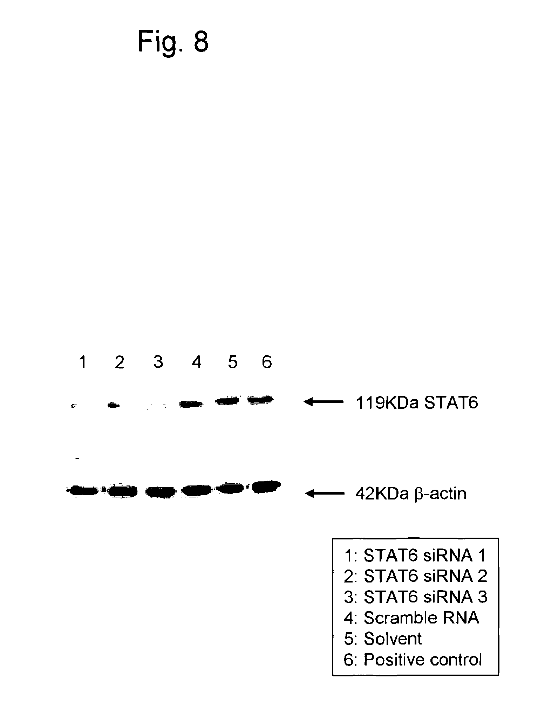

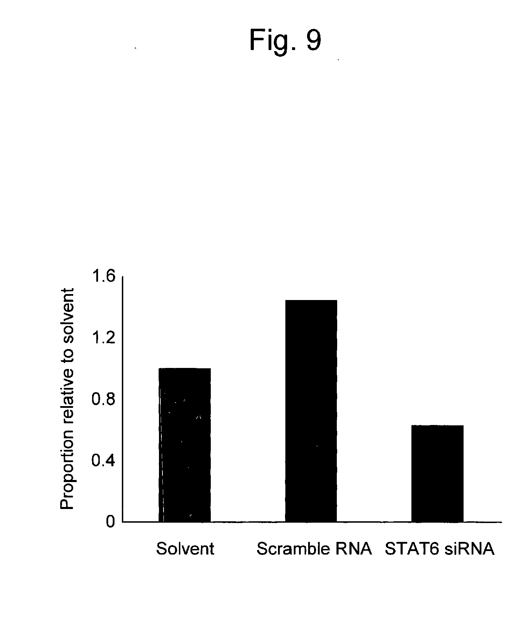

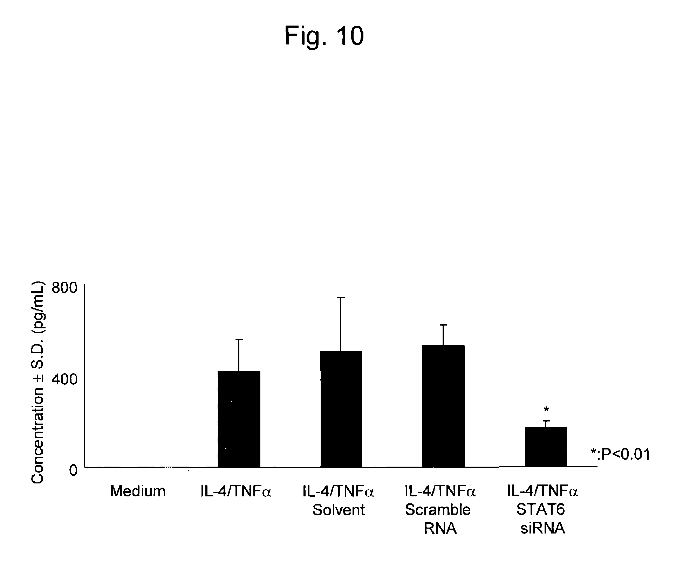

Candidate sequences for mouse STAT6 siRNA are shown below. Fig. 8 shows the results of suppression of STAT6 protein expression confirmed via Western blotting. As shown in Fig. 8, all of STAT6 siRNAs 1, 2, and 3 exhibited the effects of suppressing STAT6 protein expression. STAT6 siRNA 3 that had most efficiently suppressed STAT6 protein expression was used as STAT6 siRNA below. Fig. 9 shows the results of suppression of STAT6 mRNA expression confirmed via RT-PCR, and Fig. 10 shows the results of quantification via ELISA of the concentration of eotaxin (CCL11) produced upon IL-4 (10 ng/ml) and TNF-alpha (40 ng/ml) costimulation. As shown in Fig. 9 and Fig. 10, newly developed mouse STAT6 siRNA exhibited the effects of suppressing the expression of STAT6 mRNA and the production of eotaxin (CCL11).
Mouse STAT6 siRNA 1
5'-GCCGAGGCACCCUGUAUAUCC sense strand (SEQ ID NO: 9)
GACGGCUCCGUGGGACAUAUA-5' anti-sense strand (SEQ ID NO: 10)
Mouse STAT6 siRNA 2
5'-CCUGGUUCUGUUAAGGAUUCA sense strand (SEQ ID NO: 11)
GGGGACCAAGACAAUUCCUAA-5' anti-sense strand (SEQ ID NO: 12)
Mouse STAT6 siRNA3
5'-CGAAUGUGAUACAACUGUAUC sense strand (SEQ ID NO: 13)
GAGCUUACACUAUGUUGACAU-5' anti-sense strand (SEQ ID NO: 14)

### 6. Effects of STAT6 siRNA on contact hypersensitivity responses

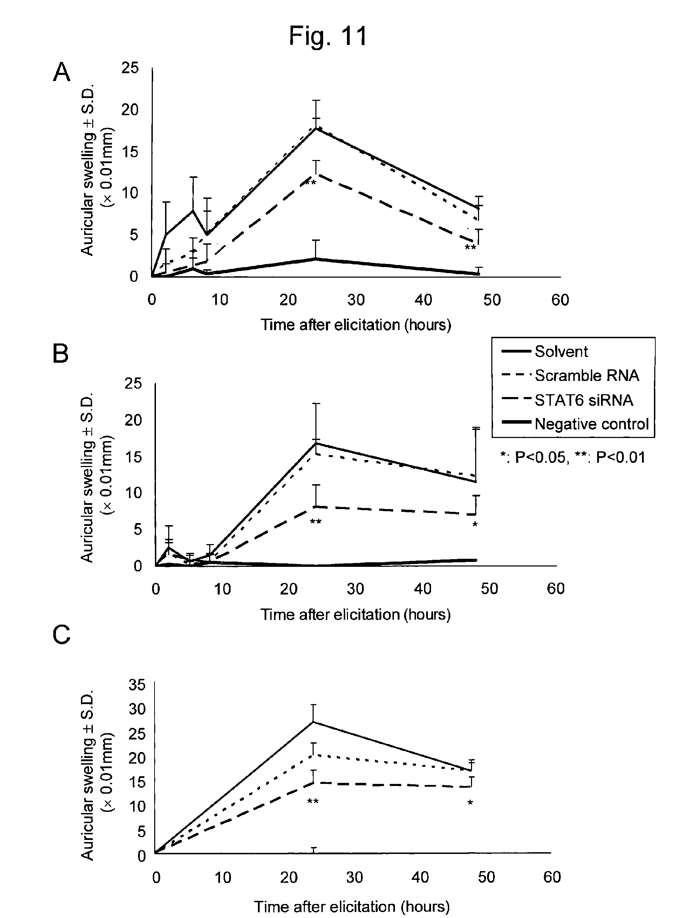

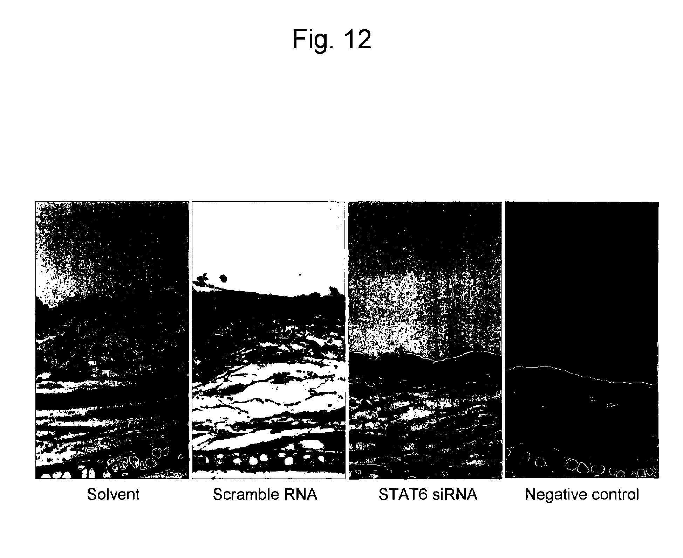

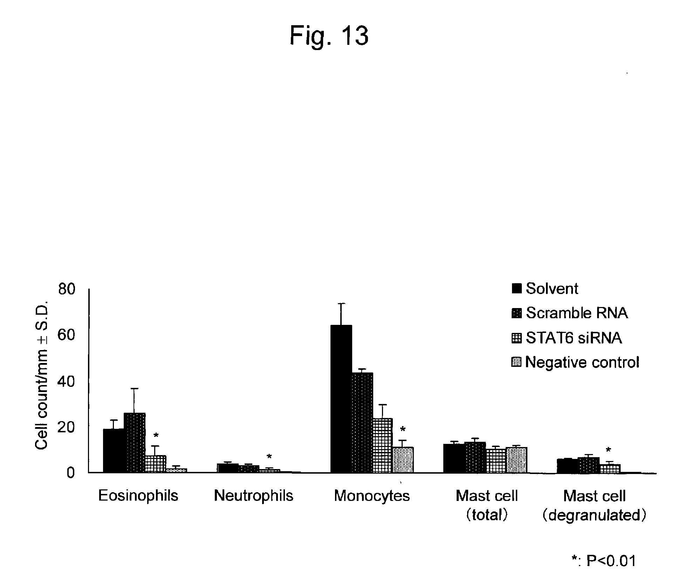

Contact hypersensitivity responses to TNCB, DNFB, and oxazolone were significantly suppressed by subcutaneous administration of STAT6 siRNA to the auricles (Fig. 11). Contact hypersensitivity responses to TNCB were histopathologically examined via Giemsa staining. As a result, significant reduction in edema and cellular infiltration was observed in the group to which STAT6 siRNA had been administered, in comparison with the control group (Fig. 12). As a result of examination of the profiles of infiltrated cells, the number of mononuclear cells, eosinophils, neutrophils, and degranulated mast cells was found to have decreased (Fig. 13).

### 7. Effects of STAT6 siRNA-containing ointment on contact hypersensitivity responses

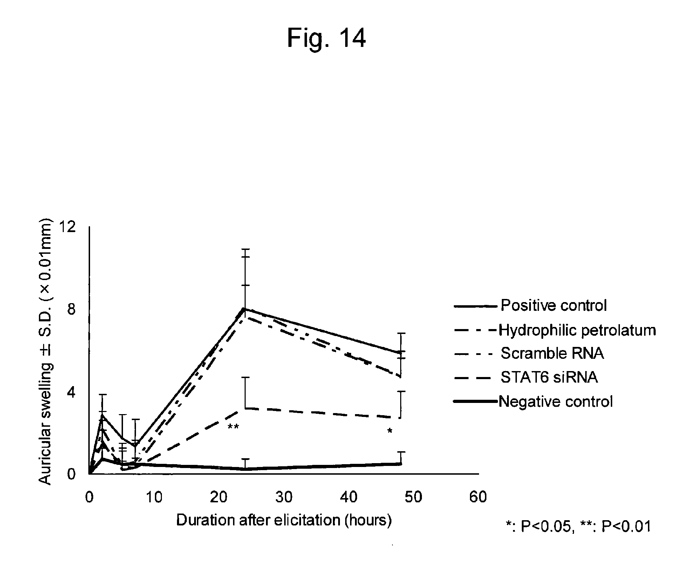

The STAT6 siRNA-containing ointment was prepared and applied to the auricles. As a result, auricular swelling was significantly suppressed in comparison with the control group (Fig. 14). Based on such results, use of STAT6 siRNA for an ointment was considered to be possible.

### 8. Effects of STAT6 siRNA on rhinitis models

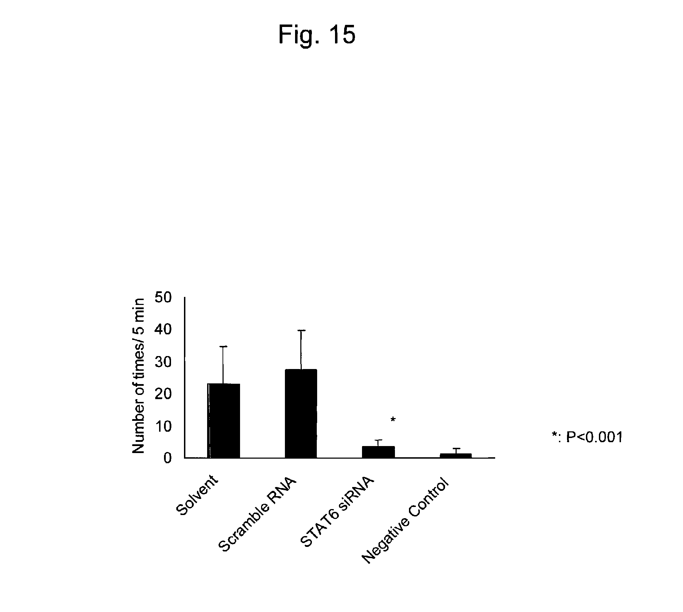

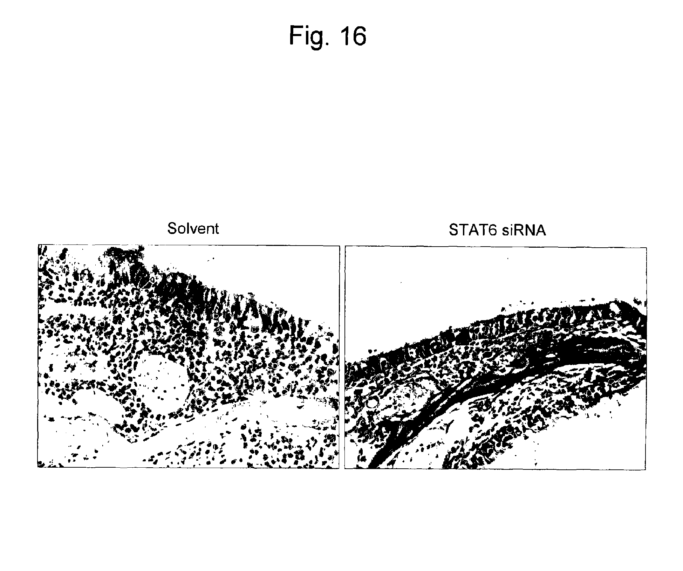

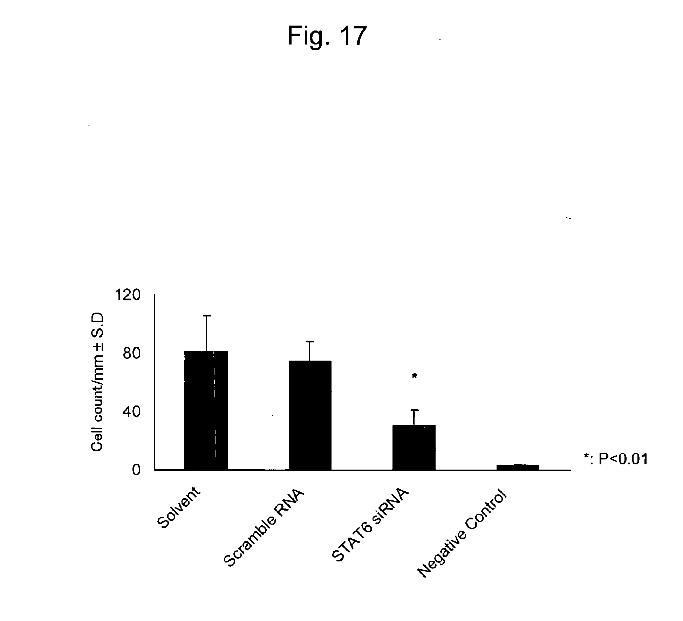

OVA was administered to the OVA-sensitized mice through the nasal cavity by inhalation successively from Day 21 to Day 27 to elicit rhinitis reactions. STAT6 siRNA dissolved in PBS was applied to the nasal cavity on Days 22, 23, and 24 (3 nmol/day), and the therapeutic effects thereof were examined. As a result, the number of times of sneezing was significantly decreased via administration of STAT6 siRNA (Fig. 15). Suppression of inflammatory reactions was histopathologically confirmed in terms of, for example, significant decrease in eosinophilic infiltration. Fig. 16 shows the results of Giemsa staining and Fig. 17 shows the number of infiltrated eosinophils.

### Industrial Applicability

Use of the dsRNA molecule of the present invention enables specific suppression of STAT6 gene expression and, in turn, enables inhibition of Th2-type cytokine and chemokine production. Thus, allergic diseases can be prevented and treated.

All publications, patents, and patent applications cited herein are incorporated herein by reference in their entirety.

### Sequence Listing Free Text

SEQ ID NOs: 5 to 14; synthetic sequences

## Claims

1. A double-stranded RNA (dsRNA) molecule targeting mRNA of the STAT6 gene composed of (a) or (b) below:
(a) a sense strand identical to a sequence comprising 15 to 50 continuous nucleotides of the nucleotide sequence resulting from substitution of thymine with uracil in the STAT6 gene sequence represented by SEQ ID NO: 1 or 3 and an antisense strand comprising a nucleotide sequence complementary to the nucleotide sequence of the sense strand; or
(b) a sense strand comprising a sequence derived from the sequence comprising at least 15 to 50 continuous nucleotides of the nucleotide sequence resulting from substitution of thymine with uracil in the STAT6 gene sequence represented by SEQ ID NO: 1 or 3 by deletion, substitution, or addition of 1 or several nucleotides and capable of hybridizing to the STAT6 gene and an antisense strand comprising a nucleotide sequence complementary to the nucleotide sequence of the sense strand.

2. The dsRNA molecule according to claim 1 composed of (c) or (d) below:
(c) a sense strand identical to a nucleotide sequence selected from the group consisting of: a sequence comprising nucleotides 3426 to 3446 of the nucleotide sequence resulting from substitution of thymine with uracil in the human STAT6 gene sequence represented by SEQ ID NO: 1; a sequence comprising nucleotides 3432 to 3452 of the nucleotide sequence resulting from substitution of thymine with uracil in the human STAT6 gene sequence represented by SEQ ID NO: 1; a sequence comprising nucleotides 259 to 279 of the nucleotide sequence resulting from substitution of thymine with uracil in the mouse STAT6 gene sequence represented by SEQ ID NO: 3; and a sequence comprising nucleotides 3026 to 3046 of the nucleotide sequence resulting from substitution of thymine with uracil in the mouse STAT6 gene sequence represented by SEQ ID NO: 3 and an antisense strand comprising a nucleotide sequence complementary to the nucleotide sequence of the sense strand; or
(d) a sense strand comprising a sequence derived from any nucleotide sequence selected from the group consisting of: a sequence comprising nucleotides 3426 to 3446 of the nucleotide sequence resulting from substitution of thymine with uracil in the human STAT6 gene sequence represented by SEQ ID NO: 1; a sequence comprising nucleotides 3432 to 3452 of the nucleotide sequence resulting from substitution of thymine with uracil in the human STAT6 gene sequence represented by SEQ ID NO: 1; a sequence comprising nucleotides 259 to 279 of the nucleotide sequence resulting from substitution of thymine with uracil in the mouse STAT6 gene sequence represented by SEQ ID NO: 3; and a sequence comprising nucleotides 3026 to 3046 of the nucleotide sequence resulting from substitution of thymine with uracil in the mouse STAT6 gene sequence represented by SEQ ID NO: 3 by deletion, substitution, or addition of 1 or several nucleotides and capable of hybridizing to the STAT6 gene sequence and an antisense strand comprising a nucleotide sequence complementary to the nucleotide sequence of the sense strand.

3. The dsRNA molecule according to claim 2, which is composed of any base pairs selected from the group consisting of: base pairs comprising a sense strand represented by SEQ ID NO: 5 and an antisense strand represented by SEQ ID NO: 6; base pairs comprising a sense strand represented by SEQ ID NO: 7 and an antisense strand represented by SEQ ID NO: 8; base pairs comprising a sense strand represented by SEQ ID NO: 9 and an antisense strand represented by SEQ ID NO: 10; base pairs comprising a sense strand represented by SEQ ID NO: 11 and an antisense strand represented by SEQ ID NO: 12; and base pairs comprising a sense strand represented by SEQ ID NO: 13 and an antisense strand represented by SEQ ID NO: 14.

4. The dsRNA molecule according to any one of claims 1 to 3, wherein the sense strand is ligated to the antisense strand via a linker molecule.

5. A vector comprising template DNA of the dsRNA molecule according to claim 4 and expressing the dsRNA molecule.

6. A pharmaceutical composition used for treatment or prevention of allergic diseases comprising 1 or a plurality of the dsRNA molecule(s) according to any one of claims 1 to 4 and capable of suppressing STAT6 gene expression.

7. A pharmaceutical composition used for treatment or prevention of allergic diseases comprising the vector according to claim 5 and capable of suppressing STAT6 gene expression.

8. The dsRNA molecule according to any one of claims 1 to 3, which has an overhanging nucleotide comprising one or a plurality of guanines (Gs) at the 5' end of the sense strand.

9. The dsRNA molecule according to claim 8, wherein the sense strand is ligated to the antisense strand via a linker molecule.

10. A vector comprising template DNA of the dsRNA molecule according to claim 9 and expressing the dsRNA molecule.

11. A pharmaceutical composition used for treatment or prevention of allergic diseases comprising 1 or a plurality of the dsRNA molecule(s) according to claim 8 or 9, which induces no interferon and/or cell damage and is capable of suppressing STAT6 gene expression.

12. A pharmaceutical composition used for treatment or prevention of allergic diseases comprising the vector according to claim 10, which induces no interferon and/or cell damage and is capable of suppressing STAT6 gene expression.

13. The pharmaceutical composition used for treatment or prevention of allergic diseases according to claim 6, 7, 11, or 12, which is administered through the nasal cavity.

14. The pharmaceutical composition used for treatment or prevention of allergic diseases according to claim 6, 7, 11, or 12, which is an ointment to be applied to the skin.
